(19) Europäisches Patentamt
European Patent Office
Office européen des brevets

(11) EP 2 945 603 B1

(12) **EUROPEAN PATENT SPECIFICATION**

(45) Date of publication and mention of the grant of the patent:
**27.03.2019 Bulletin 2019/13**

(21) Application number: **14700915.3**

(22) Date of filing: **20.01.2014**

(51) Int Cl.:
*A61K 8/41* (2006.01) *A61Q 17/04* (2006.01)
*A61K 8/35* (2006.01) *A61K 8/44* (2006.01)
*A61K 8/46* (2006.01) *A61K 8/49* (2006.01)
*A61K 8/06* (2006.01)

(86) International application number:
**PCT/EP2014/051015**

(87) International publication number:
**WO 2014/111567 (24.07.2014 Gazette 2014/30)**

(54) **COSMETIC OR DERMATOLOGICAL COMPOSITION COMPRISING A MEROCYANINE AND A UVA-SCREENING AGENT OF THE AMINO-SUBSTITUTED 2-HYDROXYBENZOPHENONE TYPE AND/OR A HYDROPHILIC ORGANIC UVA-SCREENING AGENT**

KOSMETISCHE ODER DERMATOLOGISCHE ZUSAMMENSETZUNG MIT EINEM MEROCYANIN UND EINEM UVA-SCREENING-MITTEL MIT AMINOSUBSTITUIERTEM 2-HYDROXYBENZOPHENON UND/ODER EINEM HYDROPHILEN ORGANISCHEN UVA-SCREENING-MITTEL

COMPOSITION COSMÉTIQUE OU DERMATOLOGIQUE COMPRENANT UNE MÉROCYANINE ET UN AGENT ANTI-UVA DU TYPE 2-HYDROXYBENZOPHÉNONE À SUBSTITUTION AMINO ET/OU UN AGENT ANTI-UVA ORGANIQUE HYDROPHILE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.01.2013 FR 1350485**
**21.01.2013 FR 1350495**
**21.02.2013 US 201361767342 P**

(43) Date of publication of application:
**25.11.2015 Bulletin 2015/48**

(73) Proprietor: **L'Oréal**
**75008 Paris (FR)**

(72) Inventors:
- **ROUDOT, Angelina**
**F-94270 Le Kremlin Bicêtre (FR)**
- **CANDAU, Didier**
**F-91570 Bièvres (FR)**
- **LALLORET, Florence**
**F-75014 Paris (FR)**

(74) Representative: **Le Blainvaux Bellegarde, Françoise**
**L'Oréal**
**Service DIPI**
**9 Rue Pierre Dreyfus**
**92110 Clichy (FR)**

(56) References cited:
**EP-A1- 0 370 867**
**EP-A1- 1 728 501**
**EP-A2- 1 133 980**
**WO-A1-2004/006878**
**WO-A1-2011/113718**
**WO-A1-2013/010590**
**WO-A2-2008/090066**
**WO-A2-2013/011094**
**DE-A1-102005 059 738**

- **"Use of merocyanine derivatives as UV absorbers in cosmetic formulations (I)", IP.COM JOURNAL, IP.COM INC., WEST HENRIETTA, NY, US, 23 February 2009 (2009-02-23), XP013129682, ISSN: 1533-0001**
- **"Process for producing 3-amino-2-cyclohexan-1-ylidene compounds", IP.COM JOURNAL, IP.COM INC., WEST HENRIETTA, NY, US, 29 April 2009 (2009-04-29), XP013131313, ISSN: 1533-0001**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Remarks:

The file contains technical information submitted after the application was filed and not included in this specification

## Description

The present invention relates to a cosmetic or dermatological composition comprising, in a physiologically acceptable support:

a) at least one oily phase
b) at least one merocyanine compound of formula (1) defined herein below and
c) at least one UVA-screening agent chosen from:

(i) an amino-substituted 2-hydroxybenzophenone compound, and
(ii) a hydrophilic organic UVA-screening agent; the said hydrophilic UVA-screening agent being present in an amount of from 0.6% to 15% by weight relative to the total weight of the composition.

The present invention also relates to a non-therapeutic cosmetic process for caring for and/or making up a keratin material, comprising the application to the surface of the said keratin material of at least one composition according to the invention as defined above.
The invention also relates to a non-therapeutic cosmetic process for limiting the darkening of the skin and/or the improving the colour and/or the uniformity of the complexion, comprising the application to the surface of the keratin material of at least one composition as defined previously.
The invention also relates to a non-therapeutic cosmetic process for preventing and/or treating the signs of ageing of a keratin material, comprising the application to the surface of the keratin material of at least one composition as defined previously.
It is known that UV-A rays with wavelengths of between 320 and 400 nm penetrate more deeply into the skin than UV-B rays. UV-A rays cause immediate and persistent browning of the skin. Daily exposure to UVA rays, even of short duration, under normal conditions can result in damage to the collagen fibres and the elastin, which is reflected by a modification in the microrelief of the skin, the appearance of wrinkles and uneven pigmentation (liver spots, lack of uniformity of the complexion).
Protection against UVA rays is thus necessary.
Many photoprotective compositions have been proposed to date to overcome the effects induced by UVA rays. They generally contain organic or mineral UV-screening agents, which function according to their own chemical nature and according to their own properties by absorption, reflection or scattering of the UV rays. They generally comprise mixtures of liposoluble organic screening agents and/or water-soluble UV-screening agents in combination with metal oxide pigments, such as titanium dioxide or zinc oxide.
It is also known that radiation with wavelengths of between 280 nm and 400 nm permits tanning of the human epidermis and that radiation with wavelengths of between 280 and 320 nm, known as UV-B rays, harms the development of a natural tan. Exposure is also liable to bring about a detrimental change in the biomechanical properties of the epidermis, which is reflected by the appearance of wrinkles, leading to premature ageing of the skin.
Protection against UVA and UVB rays is thus necessary. An efficient photoprotective product should protect against both UVA and UVB rays. Combinations of UV-screening agents which absorb UVA radiation and screening agents which absorb UVB radiation are generally used in antisun formulations, so as to obtain the broadest possible protection.
Many cosmetic compositions for limiting the darkening of the skin and improving the colour and the uniformity of the complexion have been proposed to date. It is well known in the field of antisun products that such compositions may be obtained by using UV-screening agents, and in particular UVB-screening agents. Certain compositions may also contain UVA-screening agents. This screening system should cover UVB protection for the purpose of limiting and controlling the neosynthesis of melanin, which promotes the overall pigmentation, but should also cover UVA protection so as to limit and control the oxidation of the already existing melanin leading to darkening of the skin colour.
However, it is extremely difficult to find a composition which contains a particular combination of UV-screening agents that would be especially suited to improving the quality of the skin as regards both the colour and its mechanical elasticity properties. This improvement is particularly sought on already pigmented skin so as not to increase the melanin pigmentary load or the structure of the melanin already present in the skin.
In point of fact, the majority of the organic UV-screening agents consist of aromatic compounds which absorb in the wavelength range between 280 and 370 nm. In addition to their power for screening out sunlight, the desired photoprotective compounds should also have good cosmetic properties, good solubility in the usual solvents and in particular in fatty substances such as oils, and also good chemical stability and good photostability alone or in combination with other UV-screening agents. They should also be colourless or at least have a colour that is cosmetically acceptable to the consumer.
One of the main drawbacks known to date is that these organic systems for screening out UVA radiation are

insufficiently effective against UVA rays and particularly against long UVA rays with wavelengths beyond 370 nm.

In this respect, a particularly advantageous family of UVA-screening agents is currently composed of amino-substituted 2-hydroxybenzophenone derivatives and especially n-hexyl 2-(4-diethylamino-2-hydroxybenzoyl)benzoate, which in fact show high intrinsic absorbing power. These amino-substituted 2-hydroxybenzophenone derivatives are known per se and their structures and syntheses are described in patent applications EP-A-1 046 391, EP 1 133 980, DE 100 12 408 and WO 2007/071 584.

n-Hexyl 2-(4-diethylamino-2-hydroxybenzoyl)benzoate is more particularly known, which is sold under the trade name Uvinul A Plus® by the company BASF. However, these compounds do not afford broad UV protection over the range from 320 to 400 nm. Specifically, they afford protection from 320 nm to 370 nm, and at best to 380 nm in large amounts, but do not in particular make it possible to obtain observable absorbance up to a wavelength of 400 nm inclusive.

Among the UVA-screening agents, hydrophilic aromatic organic molecules which absorb in the wavelength range between 320 and 370 nm are particularly advantageous. Specifically, their solubility in the aqueous phase reduces the amounts of oil needed in the formulations and thus limits the cosmetic drawbacks often caused by the presence of lipophilic UV-screening agents, such as the greasy aspect on application. They may be used especially in oil-in-water emulsions (i.e. a cosmetically and/or dermatologically acceptable support consisting of an aqueous dispersing continuous phase and of a fatty dispersed discontinuous phase) or water-in-oil emulsions (aqueous phase dispersed in a continuous fatty phase).

Among the hydrophilic organic UVA-screening agents, benzene-1,4-bis(3-methylidene-10-camphorsulfonic acid) and the various salts thereof are especially known, which are described especially in patent applications FR-A-2 528 420 and FR-A-2 639 347, which are screening agents that are already known per se ("broad-band" screening agents) with absorption maxima of between 320 and 370 nm, in particular at about 345 nm.

Among the hydrophilic organic UVA-screening agents, compounds comprising at least two benzazolyl groups bearing sulfonic groups are also known in patent application EP-A-0 669 323. They are described and prepared according to the syntheses indicated in patent US 2 463 264 and in patent application EP-A-0 669 323.

Among the hydrophilic organic UVA-screening agents, benzophenone derivatives comprising at least one sulfonic radical are also known, for instance Benzophenone-4, sold by the company BASF under the name Uvinul MS 40:

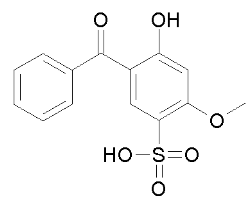

Benzophenone-5 of structure

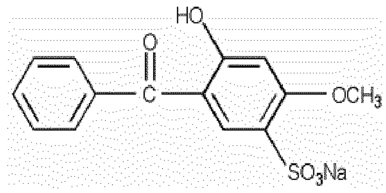

;

and Benzophenone-9, sold by the company BASF under the name Uvinul DS 49:

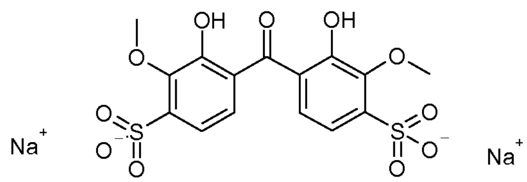

However, these compounds do not afford broad protection against UV radiation over the range from 320 to 400 nm. Specifically, they afford protection from 320 nm to 370 nm, and at best to 380 nm in large amounts, but do not in particular make it possible to obtain observable absorbance up to a wavelength of 400 nm inclusive.

Merocyanine compounds are known in patent US 4 195 999, patent application WO 2004/006 878, patent

applications WO2008/090066, WO2011/113718, WO2009/027258, WO2013/010590, WO2013/011094, WO20130/11480 and the documents IP COM JOURNAL N°000179675D published on February 23, 2009, IP COM JOURNAL N°000182396D published on April 29, IP COM JOURNAL N° 000189542D published on November 12, 2009, IP COM Journal N°IPCOM000011179D published on 03/04/2004.

Some of these compounds may show the following drawbacks :

- relatively unsatisfactory solubility in the usual solvents and in particular in fatty substances such as oils which may require a laborious formulation process and/or may result in cosmetic drawbacks such as a greasy effect on application ;
- an unsatisfactory chemical stability and/or unsatisfactory photostability
- produce a color liable to discourage the consumer from using a cosmetic or dermatological composition containing them.

The UVA and UVB screening systems consisting of some of these merocyanine screening agents as the compound Octyl-5-N,N-diethylamino-2-phenysulfonyl-2,4-pentadienoate corresponding to the compound MC172 of structure

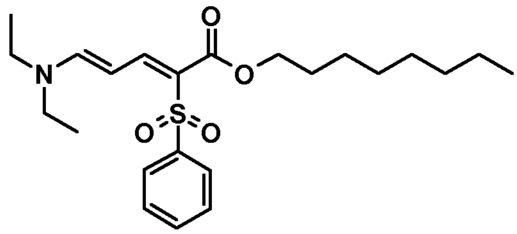

and an amino-substituted 2-hydroxybenzophenone compound or a hydrophilic UVA-screening agent do not always make it possible to afford broad UV protection over the range 280 to 400 nm and especially to obtain an observable absorbance up to a wavelength of 400 nm inclusive.

There thus remains a need to find a novel UVA-screening system based on a merocyanine compound and an amino-substituted 2-hydroxybenzophenone compound and/or a hydrophilic UVA-screening agent, which is photostable and which ensures overall protection against UVA rays from 320 to 400 nm especially having notable absorbance ranging up to a wavelength of 400 nm inclusive, in a manner that is stable over time and at high temperatures, without the drawbacks as previously defined.

The Applicant has found, surprisingly, that this objective can be achieved by combining at least one UVA-screening agent chosen from: (i) an amino-substituted 2-hydroxybenzophenone compound , (ii) a hydrophilic UVA-screening agent and (iii) their mixtures, with at least one particular merocyanine compound of formula (1) which will be defined in detail herein below.

Furthermore, the merocyanine compounds of formula (1) herein below, present surprinsingly the advantage to be significantly less colored than the merocyanine compounds as disclosed in the application WO2008/090066 as the compound MC11 also called MC03 in the application WO2009/027258.

Those discoveries form the basis of the present invention.

Thus, in accordance with one of the objects of the present invention, a cosmetic or dermatological composition is now proposed, comprising, in a physiologically acceptable support:

a) at least one oily phase
b) at least one merocyanine compound of formula (1) defined herein below and
c) at least one UVA-screening agent chosen from :

(i) an amino-substituted 2-hydroxybenzophenone compound, and
(ii) a hydrophilic organic UVA-screening; the said hydrophilic UVA-screening agent being present in an amount of from 0.6% to 15% by weight relative to the total weight of the composition.

The present invention also relates to a non-therapeutic cosmetic process for caring for and/or making up a keratin material, comprising the application to the surface of the said keratin material of at least one composition according to the invention as defined above.

The invention also relates to a non-therapeutic cosmetic process for limiting the darkening of the skin and/or improving the colour and/or the uniformity of the complexion, comprising the application to the surface of the keratin

material of at least one composition as defined previously.

The invention also relates to a non-therapeutic cosmetic process for preventing and/or treating the signs of ageing of a keratin material, comprising the application to the surface of the keratin material of at least one composition as defined previously.

Other characteristics, aspects and advantages of the invention will emerge on reading the detailed description that follows.

The term "hydrophilic organic UVA-screening agent" means any cosmetic or dermatological organic compound for screening out UVA rays in the wavelength range from 320 to 400 nm, which can be fully dissolved in molecular form in a liquid aqueous phase or which can be dissolved in colloidal form (for example in micellar form) in a liquid aqueous phase. The said hydrophilic organic UVA-screening agent is different from an amino-substituted 2-hydroxybenzophenone compound.

The expression "human keratin materials" means the skin (body, face, area around the eyes), hair, eyelashes, eyebrows, body hair, nails, lips or mucous membranes.

The term "physiologically acceptable" means compatible with the skin and/or its integuments, having a pleasant colour, odour and feel and not causing any unacceptable discomfort (stinging, tautness or redness) liable to discourage the consumer from using this composition.

The term "between X and Y" means the range of values also including the limits X and Y.

According to the invention, the term "preventing" or "prevention" means reducing the risk of occurrence or slowing down the occurrence of a given phenomenon, namely, according to the present invention, the signs of ageing of a keratin material.

## MEROCYANINES

According to the present invention, the merocyanine compounds in accordance with the invention correspond to formula (1) below, and also the E/E- or E/Z-geometrical isomer forms thereof:

(1) 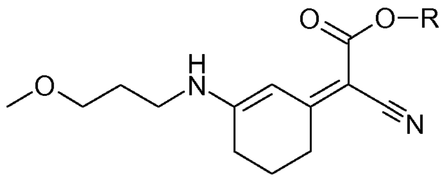

in which:

R is a $C_1$-$C_{22}$ alkyl group, a $C_2$-$C_{22}$ alkenyl group, a $C_2$-$C_{22}$ alkynyl group, a $C_3$-$C_{22}$ cycloalkyl group or a $C_3$-$C_{22}$ cycloalkenyl group, the said groups possibly being interrupted with one or more O.

The merocyanine compounds of the invention may be in their E/E- or E/Z-geometrical isomer forms.

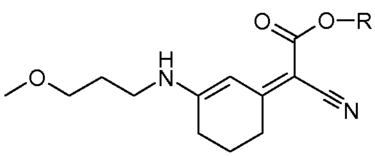

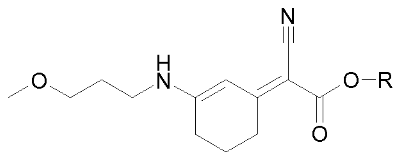

The preferential compounds of formula (1) are those in which:

R is a $C_1$-$C_{22}$ alkyl, which may be interrupted with one or more O.

Among the compounds of formula (1), use will be made more particularly of those chosen from the following compounds, and also the E/E- or E/Z- geometrical isomer forms thereof:

| | | | |
|---|---|---|---|
| 1 | 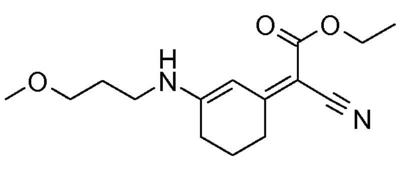 ethyl (2Z)-cyano{3-[(3-methoxypropyl)amino] cyclohex-2-en-1-ylidene}ethanoate | 4 | 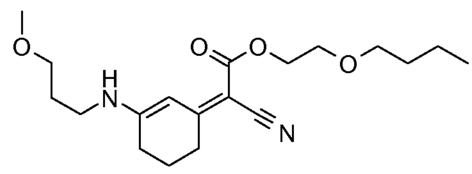 2-butoxyethyl (2Z)-cyano{3-[(3-methoxypropyl) amino]cyclohex-2-en-1-ylidene}ethanoate |

(continued)

| | | | |
|---|---|---|---|
| 2 | 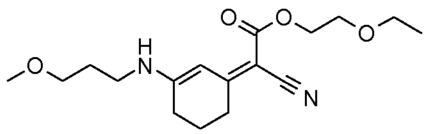<br>2-ethoxyethyl (2Z)-cyano{3-[(3-methoxypropyl) amino]cyclohex-2-en-1-ylidene}ethanoate | 5 | 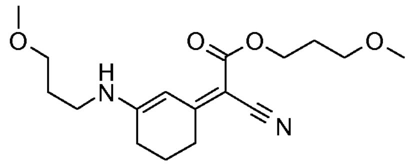<br>3-methoxypropyl (2Z)-cyano{3-[(3-methoxypropyl) amino]cyclohex-2-en-1-ylidene}ethanoate |
| 3 | 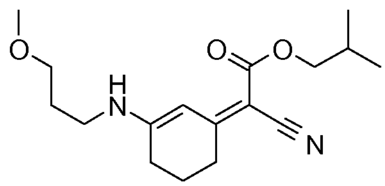<br>2-methylpropyl (2Z)-cyano{3-[(3-methoxypropyl) amino]cyclohex-2-en-1-ylidene}ethanoate | 6 | 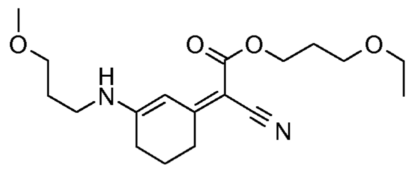<br>3-ethoxypropyl (2Z)-cyano{3-[(3-methoxypropyl) amino]cyclohex-2-en-1-ylidene}ethanoate |

According to a more particularly preferred mode of the invention, use will be made of the compound 2-ethoxyethyl (2Z)-cyano{3-[(3-methoxypropyl)amino]cyclohex-2-en-1-ylidene}ethanoate (2) in its E/Z geometrical configuration having the following structure:

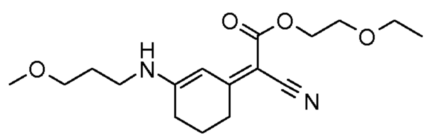

and/or in its E/E geometrical configuration having the following structure:

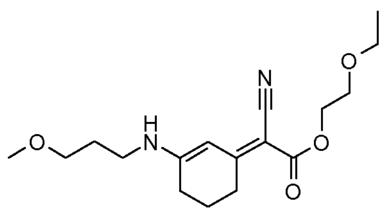

The merocyanines of formula (1) according to the invention are preferably present in the compositions according to the invention in a concentration ranging from 0.1% to 10% by weight and preferentially from 0.2% to 5% by weight relative to the total weight of the composition.

The compounds of formula (1) may be prepared according to the protocols described in Pat. Appl. WO 2007/071 582, in IP.com Journal (2009), 9(5A), 29-30 IPCOM000182396D under the title "Process for producing 3-amino-2-cyclohexan-1-ylidene compounds" and in US-A-4 749 643 on column 13, line 66 - column 14, line 57 and the references cited in this regard.

**AMINO-SUBSTITUTED 2-HYDROXYBENZOPHENONE COMPOUND**

The amino-substituted 2-hydroxybenzophenone compounds according to the invention are preferably chosen from the compounds corresponding to formula (I) below:

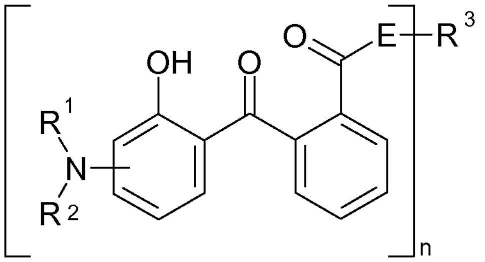

(I)

in which:

$R^1$ and $R^2$, which may be identical or different, denote a $C_1$-$C_{20}$ alkyl radical, a $C_2$-$C_{20}$ alkenyl radical, a $C_3$-$C_{10}$ cycloalkyl radical or a $C_3$-$C_{10}$ cycloalkenyl radical or form, with the nitrogen atom to which they are attached, a 5- or 6-membered ring; n is a number ranging from 1 to 4;

when n = 1, $R^3$ denotes a $C_1$-$C_{20}$ alkyl radical, a $C_1$-$C_{20}$ alkenyl radical, a $C_1$-$C_5$ hydroxyalkyl radical, a $C_6$-$C_{12}$ cyclohexyl radical, a phenyl which may be substituted with O, N or S, an aminocarbonyl radical or a $C_1$-$C_5$ alkylcarbonyl radical;

when n = 2, $R^3$ denotes an alkyl diradical, a cycloalkyl diradical, an alkenyl diradical or an aryl diradical or $R^3$ with E form a diradical of formula (II):

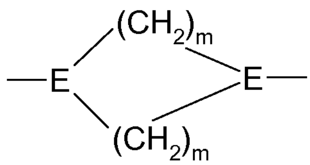

(II)

with m being a number ranging from 1 to 3;

when n = 3, $R^3$ is an alkyl triradical;

when n = 4, $R^3$ is an alkyl tetraradical;

E is -O- or -N($R^4$)- or N;

$R^4$ is hydrogen or a $C_1$-$C_5$ alkyl or $C_1$-$C_5$ hydroxyalkyl radical.

Examples of $C_1$-$C_{20}$ alkyl radicals that may be mentioned include: methyl, ethyl, n-propyl, 1-methylethyl, n-butyl, 1-methylpropyl, 2-methylpropyl, 1,1-dimethylethyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,2,2-trimethylpropyl, 1-ethyl-1-methylpropyl, 1-ethyl-2-methylpropyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonadecyl or n-eicosyl.

Examples of $C_3$-$C_{10}$ cycloalkyl radicals that may be mentioned include: cyclopropyl, cyclobutyl, cyclopentyl, cyclohexyl, cycloheptyl, 1-methylcyclopropyl, 1-ethylcyclopropyl, 1-propylcyclopropyl, 1-butylcyclopropyl, 1-pentylcyclopropyl, 1-methyl-1-butylcyclopropyl, 1,2-dimethylcyclopropyl, 1-methyl-2-ethylcyclopropyl, cyclooctyl, cyclononyl or cyclodecyl.

As $C_3$-$C_{10}$ cycloalkenyl radicals bearing one or more double bonds, mention may be made of: cyclopropenyl, cyclobutenyl, cyclopentenyl, cyclopentadienyl, cyclohexenyl, 1,3-cyclohexadienyl, 1,4-cyclohexadienyl, cycloheptenyl, cycloheptatrienyl, cyclooctenyl, 1,5-cyclooctadienyl, cyclooctatetraenyl, cyclononenyl or cyclodecenyl.

As examples of 5- or 6-membered rings formed by the radicals R1 and R2 with the nitrogen atom, mention may be made in particular of pyrrolidine or piperidine.

A compound of formula (I) with n = 1 that is most particularly preferred is n-hexyl 2-(4-diethylamino-2-hydroxybenzoyl)benzoate of formula (a):

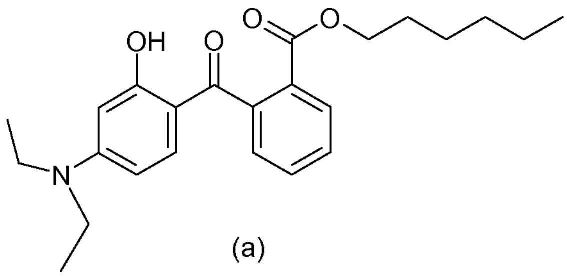

(a)

such as the product sold under the trade name Uvinul A Plus® by the company BASF.

A compound of formula (I) with n = 2 that is most particularly preferred is the derivative (2-{4-[2-(4-diethylamino-2-hydroxybenzoyl)benzoyl]piperazine-1-carbonyl}phenyl)(4-diethylamino-2-hydroxyphenyl)methanone (CAS 919803-06-8) of formula (b):

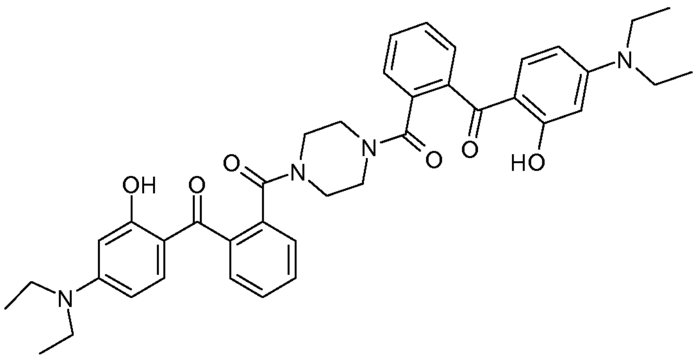

(b)

which is described in patent application WO 2007/071 584. This compound is advantageously used in micronized form (mean size of 0.02 to 2 μm), which may be obtained, for example, according to the micronization process described in patent applications GB-A-2 303 549 and EP-A-893 119, and more particularly in the form of an aqueous dispersion.

The compounds of formula (I) as defined above are known per se and their structures and syntheses are described in patent applications EP-A-1 046 391, EP 1 133 980, DE 100 12 408 and WO 2007/071 584.

The amino-substituted 2-hydroxybenzophenone compound(s) are preferably present in the compositions in accordance with the invention in contents preferably ranging from 0.01% to 10% by weight and more preferentially from 0.1% to 6% by weight relative to the total weight of the composition.

## HYDROPHILIC ORGANIC UVA-SCREENING AGENTS

Among the hydrophilic organic UVA-screening agents that may be used according to the present invention, mention may be made of:

benzene-1,4-bis(3-methylidene-10-camphorsulfonic acid) (INCI name: Terephthalylidenedicamphorsulfonic acid) and the various salts thereof, described especially in patent applications FR-A-2 528 420 and FR-A-2 639 347.

These screening agents correspond to the general formula (I) below:

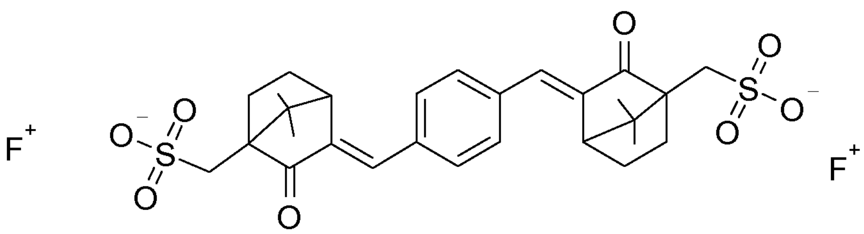

(I)

in which F denotes a hydrogen atom, an alkali metal or a radical $NH(R_1)_3^+$ in which the radicals $R_1$, which may be identical or different, denote a hydrogen atom, a $C_1$-$C_4$ alkyl or hydroxyalkyl radical or a group $M^{n+}/n$, $M^{n+}$ denoting a multivalent metal cation in which n is equal to 2 or 3 or 4, $M^{n+}$ preferably denoting a metal cation chosen from $Ca^{2+}$,

$Zn^{2+}$, $Mg^{2+}$, $Ba^{2+}$, $Al^{3+}$ and $Zr^{4+}$. It is clearly understood that the compounds of formula (I) above may give rise to the "cis-trans" isomer about one or more double bonds and that all the isomers are included in the context of the present invention.

Among the hydrophilic organic UVA-screening agents that may be used according to the present invention, mention may also be made of compounds comprising at least two benzazolyl groups bearing sulfonic groups, such as those described in patent application EP-A-0 669 323. They are described and prepared according to the syntheses indicated in patent US 2 463 264 and in patent application EP-A-0 669 323.

The compounds comprising at least two benzazolyl groups in accordance with the invention correspond to the general formula (II) below:

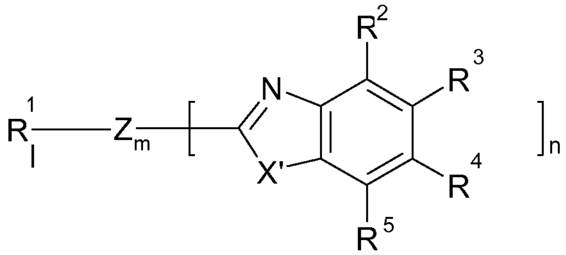

(II)

in which:

- Z represents an organic residue of valency (l + n) comprising one or more double bonds placed such that it completes the double bond system of at least two benzazolyl groups as defined within the brackets to form a fully conjugated assembly;
- X' denotes S, O or $NR^6$
- $R^1$ denotes hydrogen, $C_1$-$C_{18}$ alkyl, $C_1$-$C_4$ alkoxy, a $C_5$-$C_{15}$ aryl, a $C_2$-$C_{18}$ acyloxy, $SO_3Y$ or COOY;
- the radicals $R^2$, $R^3$, $R^4$ and $R^5$, which may be identical or different, denote a nitro group or a radical $R^1$;
- $R^6$ denotes hydrogen, a $C_1$-$C_4$ alkyl or a $C_1$-$C_4$ hydroxyalkyl;
- Y denotes hydrogen, Li, Na, K, $NH_4$, 1/2Ca, 1/2Mg, 1/3Al or a cation resulting from the neutralization of a free acid group with a nitrogenous organic base;
- m is 0 or 1;
- n is a number from 2 to 6;
- l is a number from 1 to 4;
- with the proviso that l + n does not exceed the value 6.

Among these compounds, the ones that are preferred are those for which the group Z is chosen from the group consisting of:

(a) an olefinic linear aliphatic $C_2$-$C_6$ hydrocarbon-based radical which may be interrupted with a $C_5$-$C_{12}$ aryl group or a $C_4$-$C_{10}$ heteroaryl group, chosen in particular from the following groups:
-CH=CH-, -CH=CH-CH=CH- or

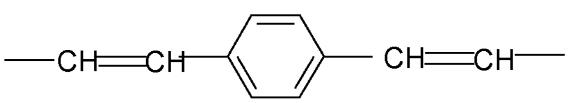

(b) a $C_5$-$C_{15}$ aryl group which may be interrupted with an olefinic linear aliphatic $C_2$-$C_6$ hydrocarbon-based radical chosen in particular from the following groups:

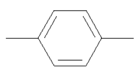

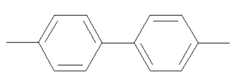

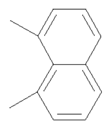

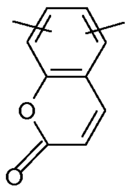

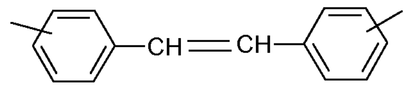

(c) a $C_3$-$C_{10}$ heteroaryl residue chosen in particular from the following groups:

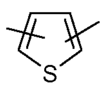
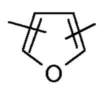
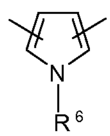
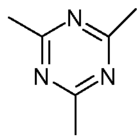

in which $R^6$ has the same meaning indicated above; the said radicals Z as defined in paragraphs (a), (b) and (c) possibly being substituted with $C_1$-$C_6$ alkyl, $C_1$-$C_6$ alkoxy, phenoxy, hydroxyl, methylenedioxy or amino radicals optionally substituted with one or two $C_1$-$C_5$ alkyl radicals.

Preferably, the compounds of formula (II) comprise, per molecule, 1, 3 or 4 groups $SO_3Y$.

As examples of compounds of formula (II) that may be used, mention may be made of the compounds of formulae (a) to (j) and having the following structure, and also the salts thereof:

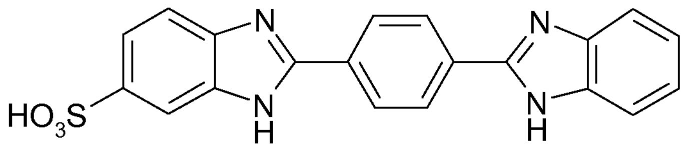
(a)

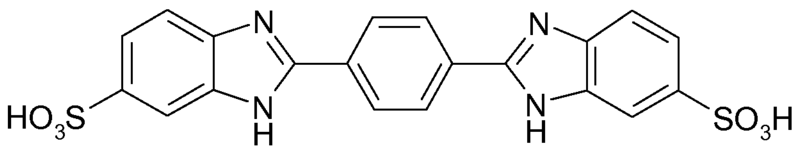
(b)

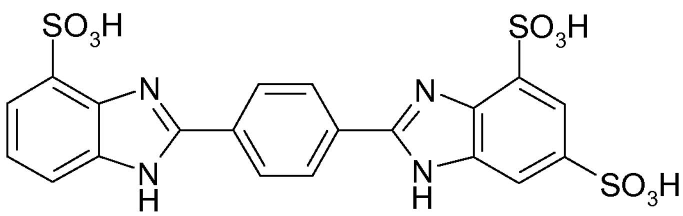
(c)

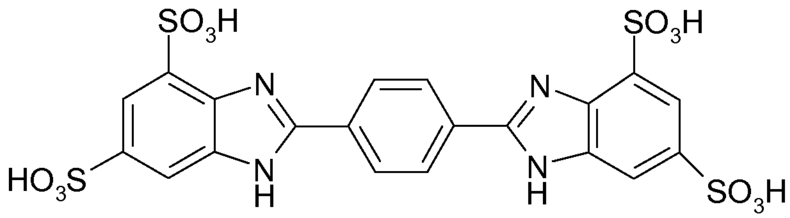

(d)

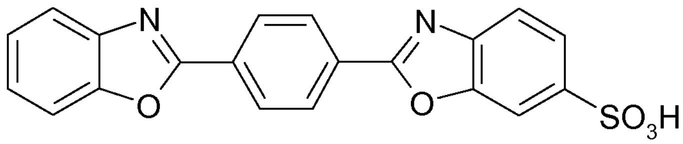

(e)

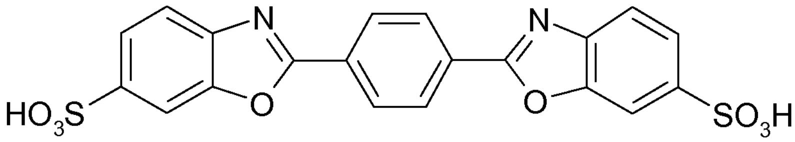

(f)

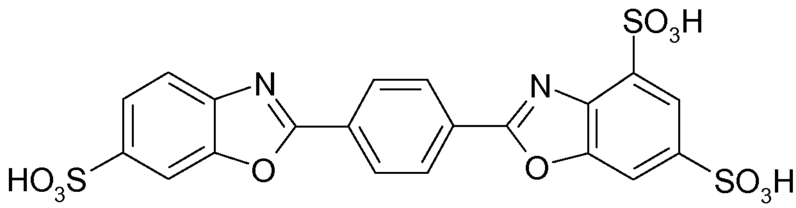

(g)

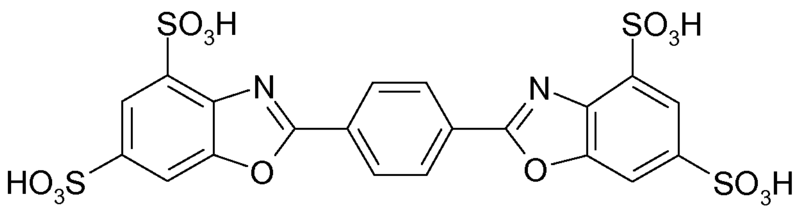

(h)

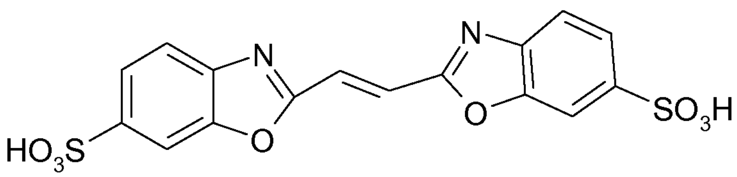

(i)

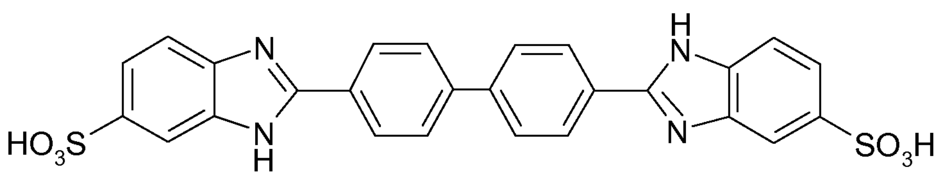

(j)

Among all these compounds, preference will be given most particularly to 1,4-bis(benzimidazolyl)phenylene-3,3',5,5'-tetrasulfonic acid (INCI name: Disodium Phenyl Dibenzimidazole Tetrasulfonate) (compound (d)) or one of the salts thereof having the following structure, sold under the name Neo Heliopan AP® by the company Symrise:

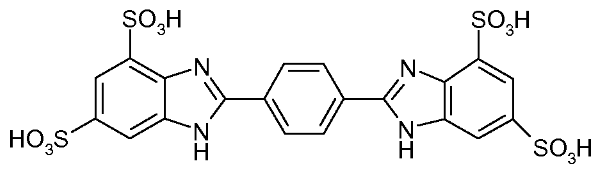

Among the hydrophilic organic UVA-screening agents that may be used according to the present invention, mention may also be made of benzophenone compounds comprising at least one sulfonic acid function, for instance the following compounds:

Benzophenone-4, sold by the company BASF under the name Uvinul MS40®:

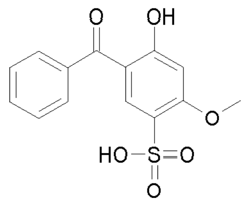

Benzophenone-5 of structure

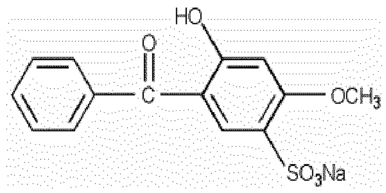

Benzophenone-9, sold by the company BASF under the name Uvinul DS49®:

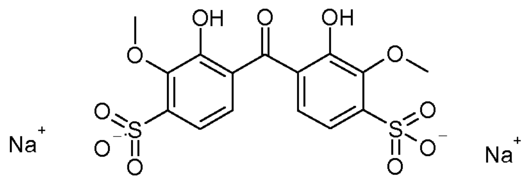

Among the hydrophilic organic UVA-screening agents, use will be made more particularly of benzene-1,4-bis(3-methylidene-10-camphorsulfonic acid) and the various salts thereof (INCI name: terephthalylidenedicamphorsulfonic acid) manufactured by the company Chimex under the trade name Mexoryl SX®.
The hydrophilic organic UVA-screening agent(s) in accordance with the invention are present in the compositions according to the invention in an active material concentration ranging from 0.6% to 15% and preferably from 1% to 10% by weight relative to the total weight of the composition.

**OILY PHASE**

The compositions in accordance with the invention comprise at least one oily phase.
For the purposes of the invention, the term "oily phase" means a phase comprising at least one oil and all of the liposoluble and lipophilic ingredients and the fatty substances used for the formulation of the compositions of the invention.
The term "oil" means any fatty substance which is in liquid form at room temperature (20 - 25°C) and at atmospheric pressure (760 mmHg).
An oil that is suitable for use in the invention may be volatile or non-volatile.
An oil that is suitable for use in the invention may be chosen from hydrocarbon-based oils, silicone oils and fluoro oils, and mixtures thereof.

A hydrocarbon-based oil that is suitable for use in the invention may be an animal hydrocarbon-based oil, a plant hydrocarbon-based oil, a mineral hydrocarbon-based oil or a synthetic hydrocarbon-based oil.

An oil that is suitable for use in the invention may be advantageously chosen from mineral hydrocarbon-based oils, plant hydrocarbon-based oils, synthetic hydrocarbon-based oils and silicone oils, and mixtures thereof.

For the purposes of the present invention, the term "silicone oil" means an oil comprising at least one silicon atom, and especially at least one Si-O group.

The term "hydrocarbon-based oil" means an oil comprising mainly hydrogen and carbon atoms.

The term "fluoro oil" means an oil comprising at least one fluorine atom.

A hydrocarbon-based oil that is suitable for use in the invention may also optionally comprise oxygen, nitrogen, sulfur and/or phosphorus atoms, for example in the form of hydroxyl, amine, amide, ester, ether or acid groups, and in particular in the form of hydroxyl, ester, ether or acid groups.

The oily phase generally comprises, in addition to the lipophilic UV-screening agent(s), at least one volatile or non-volatile hydrocarbon-based oil and/or one volatile and/or non-volatile silicone oil.

For the purposes of the invention, the term "volatile oil" means an oil that is capable of evaporating on contact with the skin or the keratin fibre in less than one hour, at room temperature and atmospheric pressure. The volatile oil(s) of the invention are volatile cosmetic oils which are liquid at room temperature and which have a non-zero vapour pressure, at room temperature and atmospheric pressure, ranging in particular from 0.13 Pa to 40 000 Pa ($10^{-3}$ to 300 mmHg), in particular ranging from 1.3 Pa to 13 000 Pa (0.01 to 100 mmHg) and more particularly ranging from 1.3 Pa to 1300 Pa (0.01 to 10 mmHg).

The term "non-volatile oil" means an oil which remains on the skin or the keratin fibre, at room temperature and atmospheric pressure, for at least several hours and which in particular has a vapour pressure of less than $10^{-3}$ mmHg (0.13 Pa).

Hydrocarbon-based oils

As non-volatile hydrocarbon-based oils that may be used according to the invention, mention may be made especially of:

(i) hydrocarbon-based oils of plant origin, such as glyceride triesters, which are generally triesters of fatty acids and of glycerol, the fatty acids of which can have varied chain lengths from $C_4$ to $C_{24}$, it being possible for these chains to be saturated or unsaturated and linear or branched; these oils are in particular wheatgerm oil, sunflower oil, grape seed oil, sesame oil, corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin oil, marrow oil, blackcurrant oil, evening primrose oil, millet oil, barley oil, quinoa oil, rye oil, safflower oil, candlenut oil, passionflower oil and musk rose oil; or also caprylic/capric acid triglycerides, such as those sold by Stéarineries Dubois or those sold under the names Miglyol 810®, 812® and 818® by Dynamit Nobel,

(ii) synthetic ethers containing from 10 to 40 carbon atoms;

(iii) linear or branched hydrocarbons of mineral or synthetic origin, such as petroleum jelly, polydecenes, hydrogenated polyisobutene such as Parleam, and squalane, and mixtures thereof;

(iv) synthetic esters, for instance oils of formula RCOOR' in which R represents a linear or branched fatty acid residue containing from 1 to 40 carbon atoms and R' represents a hydrocarbon-based chain that is especially branched, containing from 1 to 40 carbon atoms, on condition that
R + R' is ≥10, for instance purcellin oil (cetostearyl octanoate), isopropyl myristate, isopropyl palmitate, $C_{12}$-$C_{15}$ alkyl benzoate, such as the product sold under the trade name Finsolv TN® or Witconol TN® by Witco or Tegosoft TN® by Evonik Goldschmidt, 2-ethylphenyl benzoate, such as the commercial product sold under the name X-Tend 226® by ISP, isopropyl lanolate, hexyl laurate, diisopropyl adipate, isononyl isononanoate, oleyl erucate, 2-ethylhexyl palmitate, isostearyl isostearate, diisopropyl sebacate, such as the product sold under the name of "Dub Dis" by Stearinerie Dubois, octanoates, decanoates or ricinoleates of alcohols or polyalcohols, such as propylene glycol dioctanoate; hydroxylated esters, such as isostearyl lactate or diisostearyl malate; and pentaerythritol esters; citrates or tartrates, such as di(linear $C_{12}$-$C_{13}$ alkyl) tartrates, such as those sold under the name Cosmacol ETI® by Enichem Augusta Industriale, and also di(linear $C_{14}$-$C_{15}$ alkyl) tartrates, such as those sold under the name Cosmacol ETL® by the same company; or acetates;

(v) fatty alcohols that are liquid at room temperature, containing a branched and/or unsaturated carbon-based chain containing from 12 to 26 carbon atoms, for instance octyldodecanol, isostearyl alcohol, oleyl alcohol, 2-hexyldecanol,

2-butyloctanol or 2-undecylpentadecanol;

(vi) higher fatty acids, such as oleic acid, linoleic acid or linolenic acid;

(vii) carbonates such as dicaprylyl carbonate, for instance the product sold under the name Cetiol CC® by the company Cognis;

(viii) fatty amides, such as isopropyl N-lauroyl sarcosinate, such as the product sold under the trade name Eldew SL205® from Ajinomoto;
and mixtures thereof.

Among the non-volatile hydrocarbon-based oils that may be used according to the invention, preference will be given more particularly to glyceride triesters and in particular to caprylic/capric acid triglycerides, synthetic esters and in particular isononyl isononanoate, oleyl erucate, $C_{12}$-$C_{15}$ alkyl benzoate, 2-ethylphenyl benzoate and fatty alcohols, in particular octyldodecanol.

As volatile hydrocarbon-based oils that may be used according to the invention, mention may be made especially of hydrocarbon-based oils containing from 8 to 16 carbon atoms and in particular of branched $C_8$-$C_{16}$ alkanes, such as $C_8$-$C_{16}$ isoalkanes of petroleum origin (also known as isoparaffins), such as isododecane (also known as 2,2,4,4,6-pentamethylheptane), isodecane or isohexadecane, the oils sold under the Isopar or Permethyl trade names, branched $C_8$-$C_{16}$ esters, isohexyl neopentanoate, and mixtures thereof.

Mention may also be made of the alkanes described in the Cognis patent applications WO 2007/068 371 or WO 2008/155 059 (mixtures of distinct alkanes differing by at least one carbon). These alkanes are obtained from fatty alcohols, which are themselves obtained from coconut or palm oil. Mention may be made of the mixtures of n-undecane ($C_{11}$) and n-tridecane ($C_{13}$) obtained in Examples 1 and 2 of patent application WO 2008/155 059 from the company Cognis. Mention may also be made of n-dodecane ($C_{12}$) and n-tetradecane ($C_{14}$), sold by Sasol under the respective references Parafol 12-97® and Parafol 14-97®, and also mixtures thereof.

Other volatile hydrocarbon-based oils, for instance petroleum distillates, especially those sold under the name Shell Solt® by the company Shell, may also be used. According to one embodiment, the volatile solvent is chosen from volatile hydrocarbon-based oils containing from 8 to 16 carbon atoms, and mixtures thereof.

<u>b) Silicone oils</u>

The non-volatile silicone oils may be chosen in particular from non-volatile polydimethylsiloxanes (PDMSs), polydimethylsiloxanes comprising alkyl or alkoxy groups which are pendent and/or at the end of the silicone chain, which groups each contain from 2 to 24 carbon atoms, or phenyl silicones, such as phenyl trimethicones, phenyl dimethicones, phenyl(trimethylsiloxy)diphenylsiloxanes, diphenyl dimethicones, diphenyl(methyldiphenyl)trisiloxanes or (2-phenylethyl)trimethylsiloxysilicates.

Examples of volatile silicone oils that may be mentioned include volatile linear or cyclic silicone oils, especially those with a viscosity ≤ 8 centistokes ($8 \times 10^{-6}$ $m^2/s$) and especially containing from 2 to 7 silicon atoms, these silicones optionally comprising alkyl or alkoxy groups containing from 1 to 10 carbon atoms. As volatile silicone oils that may be used in the invention, mention may be made especially of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

Mention may also be made of the volatile linear alkyltrisiloxane oils of general formula (I):

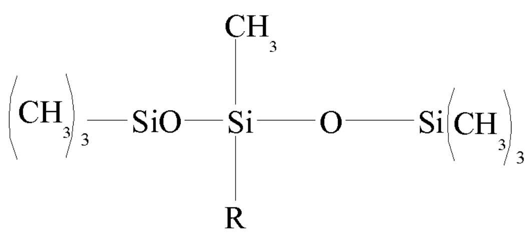

in which R represents an alkyl group comprising from 2 to 4 carbon atoms, one or more hydrogen atoms of which may be replaced with a fluorine or chlorine atom.

Among the oils of general formula (I) that may be mentioned are:

3-butyl-1,1,1,3,5,5,5-heptamethyltrisiloxane,
3-propyl-1,1,1,3,5,5,5-heptamethyltrisiloxane, and
3-ethyl-1,1,1,3,5,5,5-heptamethyltrisiloxane,

corresponding to the oils of formula (I) for which R is, respectively, a butyl group, a propyl group or an ethyl group.

Fluoro oils

Use may also be made of volatile fluoro oils, such as nonafluoromethoxybutane, decafluoropentane, tetradecafluorohexane, dodecafluoropentane, and mixtures thereof.
An oily phase according to the invention may also comprise other fatty substances, mixed with or dissolved in the oil.
Another fatty substance that may be present in the oily phase may be, for example:

- a fatty acid chosen from fatty acids comprising from 8 to 30 carbon atoms, such as stearic acid, lauric acid, palmitic acid and oleic acid;
- a wax chosen from waxes such as lanolin, beeswax, carnauba or candelilla wax, paraffin waxes, lignite waxes, microcrystalline waxes, ceresin or ozokerite, or synthetic waxes, such as polyethylene waxes or Fischer-Tropsch waxes;
- a gum chosen from silicone gums (dimethiconol);
- a pasty compound, such as polymeric or non-polymeric silicone compounds, esters of a glycerol oligomer, arachidyl propionate, fatty acid triglycerides and derivatives thereof;
- and mixtures thereof.

Preferentially, the overall oily phase, including all the lipophilic substances of the composition capable of being dissolved in this same phase, represents from 5% to 95% by weight and preferentially from 10% to 80% by weight, relative to the total weight of the composition.

**AQUEOUS PHASE**

The compositions according to the invention may also comprise at least one aqueous phase.
The aqueous phase comprises water and optionally other water-soluble or water-miscible organic solvents.
An aqueous phase that is suitable for use in the invention may comprise, for example, a water chosen from a natural spring water, such as water from La Roche-Posay, water from Vittel or waters from Vichy, or a floral water.
The water-soluble or water-miscible solvents that are suitable for use in the invention comprise short-chain monoalcohols, for example $C_1$-$C_4$ monoalcohols, such as ethanol or isopropanol; diols or polyols, such as ethylene glycol, 1,2-propylene glycol, 1,3-butylene glycol, hexylene glycol, diethylene glycol, dipropylene glycol, 2-ethoxyethanol, diethylene glycol monomethyl ether, triethylene glycol monomethyl ether, glycerol and sorbitol, and mixtures thereof.
According to a preferred embodiment, use may more particularly be made of ethanol, propylene glycol, glycerol, and mixtures thereof.
According to a specific form of the invention, the overall aqueous phase, including all the hydrophilic substances of the composition capable of being dissolved in this same phase, represents from 5% to 95% by weight and preferentially from 10% to 80% by weight, with respect to the total weight of the composition.

**ADDITIVES**

**a) Additional UV-screening agents**

The compositions according to the invention may also contain one or more additional UV-screening agents chosen from hydrophilic, lipophilic or insoluble organic UV-screening agents and/or one or more mineral pigments. It will preferentially consist of at least one hydrophilic, lipophilic or insoluble organic UV-screening agent.
The term "hydrophilic UV-screening agent" means any cosmetic or dermatological organic or mineral compound for screening out UV radiation, which can be fully dissolved in molecular form in a liquid aqueous phase or which can be dissolved in colloidal form (for example in micellar form) in a liquid aqueous phase.
The term "lipophilic screening agent" means any cosmetic or dermatological organic or mineral compound for screening out UV radiation, which can be fully dissolved in molecular state in a liquid fatty phase or which can be dissolved in colloidal form (for example in micellar form) in a liquid fatty phase.
The term "insoluble UV-screening agent" means any cosmetic or dermatological organic or mineral compound

for screening out UV radiation which has a solubility in water of less than 0.5% by weight and a solubility of less than 0.5% by weight in the majority of organic solvents such as liquid paraffin, fatty alcohol benzoates and fatty acid triglycerides, for example Miglyol® 812 sold by the company Dynamit Nobel. This solubility, determined at 70°C, is defined as the amount of product in solution in the solvent at equilibrium with an excess of solid in suspension after returning to room temperature. It may be readily evaluated in the laboratory.

The compositions according to the invention may also contain one or more additional UV-screening agents chosen from hydrophilic, lipophilic or insoluble organic UV-screening agents other than the hydroxyaminobenzophenone compounds and the hydrophilic UVA-screening agents and/or one or more mineral pigments. It will preferentially consist of at least one hydrophilic, lipophilic or insoluble organic UV-screening agent.

The additional organic UV-screening agents are chosen in particular from cinnamic compounds; anthranilate compounds; salicylic compounds; benzylidenecamphor compounds; benzophenone compounds; β,β-diphenylacrylate compounds; triazine compounds; benzotriazole compounds; benzalmalonate compounds, in particular those cited in patent US 5 624 663; benzimidazole derivatives; imidazoline compounds; bis-benzazolyl compounds, as described in patents EP 669 323 and US 2 463 264; p-aminobenzoic (PABA) compounds; methylenebis(hydroxyphenylbenzotriazole) compounds, as described in patent applications US 5 237 071, US 5 166 355, GB 2 303 549, DE 197 26 184 and EP 893 119; benzoxazole compounds, as described in patent applications EP 0 832 642, EP 1 027 883, EP 1 300 137 and DE 101 62 844; screening polymers and screening silicones, such as those described in particular in patent application WO 93/04665; α-alkylstyrene-based dimers, such as those described in patent application DE 198 55 649; 4,4-diarylbutadiene compounds, as described in patent applications EP 0 967 200, DE 197 46 654, DE 197 55 649, EP-A-1 008 586, EP 1 133 980 and EP 133 981, and mixtures thereof.

As examples of organic photoprotective agents, mention may be made of those denoted hereinbelow under their INCI name:

<u>Cinnamic compounds:</u>

Ethylhexyl Methoxycinnamate, sold in particular under the trade name Parsol MCX® by DSM Nutritional Products,
Isopropyl Methoxycinnamate,
Isoamyl p-methoxycinnamate sold under the trade name Neo Heliopan E 1000® by Symrise,
DEA Methoxycinnamate,
Diisopropyl Methyl Cinnamate,
Glyceryl Ethylhexanoate Dimethoxycinnamate.

<u>para-Aminobenzoic compounds:</u>

PABA,
Ethyl PABA,
Ethyl Dihydroxypropyl PABA,
Ethylhexyl Dimethyl PABA, sold especially under the name Escalol 507® by ISP, Glyceryl PABA,
PEG-25 PABA, sold under the name Uvinul P 25® by BASF.

<u>Salicylic compounds:</u>

Homosalate, sold under the name Eusolex HMS® by Rona/EM Industries, Ethylhexyl Salicylate, sold under the name Neo Heliopan OS® by Symrise, Dipropylene Glycol Salicylate, sold under the name Dipsal® by Scher, TEA Salicylate, sold under the name Neo Heliopan TS® by Symrise.

<u>β,β-Diphenylacrylate compounds:</u>

Octocrylene, sold in particular under the trade name Uvinul N 539® by BASF, Etocrylene, sold in particular under the trade name Uvinul N 35® by BASF.

<u>Benzophenone compounds:</u>

Benzophenone-1, sold under the trade name Uvinul 400® by BASF,
Benzophenone-2, sold under the trade name Uvinul D50® by BASF,
Benzophenone-3 or Oxybenzone, sold under the trade name Uvinul M40® by BASF,
Benzophenone-6, sold under the trade name Helisorb 11® by Norquay,

Benzophenone-8, sold under the trade name Spectra-Sorb UV-24® by American Cyanamid,
Benzophenone-12.

Benzylidenecamphor compounds:

3-Benzylidenecamphor, manufactured under the name Mexoryl SD® by Chimex, 4-Methylbenzylidenecamphor, sold under the name Eusolex 6300® by Merck, Polyacrylamidomethylbenzylidenecamphor, manufactured under the name Mexoryl SW® by Chimex.

Phenylbenzimidazole compounds:

Phenylbenzimidazolesulfonic acid, sold in particular under the trade name Eusolex 232® by Merck.

Phenylbenzotriazole compounds:

Drometrizole Trisiloxane, sold under the name Silatrizole® by Rhodia Chimie.

Methylenebis(hydroxyphenylbenzotriazole) compounds:

Methylenebis(benzotriazolyl)tetramethylbutylphenol especially in solid form, for instance the product sold under the trade name Mixxim BB/100® by Fairmount Chemical, or in the form of an aqueous dispersion of micronized particles with a mean particle size ranging from 0.01 to 5 μm, more preferentially from 0.01 to 2 μm and more particularly from 0.020 to 2 μm, with at least one alkylpolyglycoside surfactant having the structure $C_nH_{2n+1}O(C_6H_{10}O_5)_xH$, in which n is an integer from 8 to 16 and x is the mean degree of polymerization of the $(C_6H_{10}O_5)$ unit and ranges from 1.4 to 1.6, as described in patent GB-A-2 303 549, sold especially under the trade name Tinosorb M® by BASF, or in the form of an aqueous dispersion of micronized particles with a mean particle size ranging from 0.02 to 2 μm, more preferentially from 0.01 to 1.5 μm and more particularly from 0.02 to 1 μm, in the presence of at least one polyglyceryl mono($C_8$-$C_{20}$)alkyl ester with a degree of glycerol polymerization of at least 5, such as the aqueous dispersions described in patent application WO 2009/063 392.

Triazine compounds:

- Bis-Ethylhexyloxyphenol Methoxyphenyl Triazine, sold under the trade name Tinosorb S® by BASF,
- Ethylhexyl Triazone, sold in particular under the trade name Uvinul T150® by BASF,
- Diethylhexyl Butamido Triazone, sold under the trade name Uvasorb HEB® by Sigma 3V,
  2,4,6-Tris(dineopentyl 4'-aminobenzalmalonate)-s-triazine,
  2,4,6-Tris(diisobutyl 4'-aminobenzalmalonate)-s-triazine,
  2,4-Bis(n-butyl 4'-aminobenzoate)-6-(aminopropyltrisiloxane)-s-triazine,
  2,4-Bis(dineopentyl 4'-aminobenzalmalonate)-6-(n-butyl 4'-aminobenzoate)-s-triazine,
- symmetrical triazine screening agents substituted with naphthalenyl groups or polyphenyl groups described in patent US 6 225 467, patent application WO 2004/085 412 (see compounds 6 and 9) or the document "Symmetrical Triazine Derivatives", IP.COM IPCOM000031257 Journal, INC, West Henrietta, NY, US (20 September 2004), in particular 2,4,6-tris(diphenyl)triazine and 2,4,6-tris(terphenyl)triazine, which is also mentioned in patent applications WO 06/035 000, WO 06/034 982, WO 06/034 991, WO 06/035 007, WO 2006/034 992 and WO 2006/034 985, these compounds advantageously being used in micronized form (mean particle size of 0.02 to 3 μm), which may be obtained, for example, according to the micronization process described in patent applications GB-A-2 303 549 and EP-A-893 119, and in particular in aqueous dispersion;
- silicone triazines substituted with two aminobenzoate groups, as described in patent EP 0 841 341, in particular 2,4-bis(n-butyl 4'-aminobenzalmalonate)-6-[(3-{1,3,3,3-tetramethyl-1-[(trimethylsilyl)oxy]disiloxanyl}propyl)amino]-s-triazine.

Anthranilic compounds:

Menthyl anthranilate, sold under the trade name Neo Heliopan MA® by Symrise.

Imidazoline compounds:

Ethylhexyl dimethoxybenzylidene dioxoimidazoline propionate.

Benzalmalonate compounds:

Polyorganosiloxane bearing benzalmalonate functions, such as Polysilicone-15, sold under the trade name Parsol SLX® by Hoffmann-LaRoche.

4,4-Diarylbutadiene compounds:

1,1-Dicarboxy(2,2'-dimethylpropyl)-4,4-diphenylbutadiene.

Benzoxazole compounds:

2,4-Bis[5-(1,1-dimethylpropyl)benzoxazol-2-yl(4-phenyl)imino]-6-(2-ethylhexyl)imino-1,3,5-triazine sold under the name Uvasorb K2A® by Sigma 3V.

The preferred organic screening agents are chosen from:

Ethylhexyl methoxycinnamate,
Ethylhexyl salicylate,
Homosalate,
Octocrylene,
Phenylbenzimidazole sulfonic acid,
Benzophenone-3,
4-Methylbenzylidenecamphor,
Methylenebis(benzotriazolyl)tetramethylbutylphenol,
Bis(ethylhexyloxyphenol)methoxyphenyltriazine,
Ethylhexyl Triazone,
Diethylhexyl Butamido Triazone,
2,4,6-Tris(dineopentyl 4'-aminobenzalmalonate)-s-triazine,
2,4,6-Tris(diisobutyl 4'-aminobenzalmalonate)-s-triazine,
2,4-Bis(n-butyl 4'-aminobenzoate)-6-(aminopropyltrisiloxane)-s-triazine,
2,4-Bis(dineopentyl 4'-aminobenzalmalonate)-6-(n-butyl 4'-aminobenzoate)-s-triazine,
2,4-Bis(n-butyl 4'-aminobenzalmalonate)-6-[(3-{1,3,3,3-tetramethyl-1-[(trimethylsilyl)oxy]disiloxanyl}propyl)amino]-s-triazine,
2,4,6-Tris(diphenyl)triazine,
2,4,6-Tris(terphenyl)triazine,
Drometrizole Trisiloxane,
Polysilicone-15,
1,1-Dicarboxy(2,2'-dimethylpropyl)-4,4-diphenylbutadiene,
2,4-Bis[4-[5-(1,1-dimethylpropyl)benzoxazol-2-yl]phenylimino]-6-[(2-ethylhexyl)imino]-1,3,5-triazine,
and mixtures thereof.

The particularly preferred organic screening agents are chosen from: Ethylhexyl salicylate,
Homosalate,
Octocrylene,
Bis(ethylhexyloxyphenol)methoxyphenyltriazine,
Ethylhexyl Triazone,
Diethylhexyl Butamido Triazone,
2,4-Bis(n-butyl 4'-aminobenzalmalonate)-6-[(3-{1,3,3,3-tetramethyl-1-[(trimethylsilyl)oxy]disiloxanyl}propyl)amino]-s-triazine,
Drometrizole Trisiloxane,
and mixtures thereof.

The mineral UV-screening agents used in accordance with the present invention are metal oxide pigments. More preferentially, the mineral UV-screening agents of the invention are metal oxide particles with a mean elementary particle size of less than or equal to 0.5 μm, more preferentially between 0.005 and 0.5 μm, even more preferentially between 0.01 and 0.2 μm, better still between 0.01 and 0.1 μm and more particularly preferentially between 0.015 and

0.05 μm.

They may be selected in particular from titanium oxide, zinc oxide, iron oxide, zirconium oxide and cerium oxide, or mixtures thereof.

Such coated or uncoated metal oxide pigments are described in particular in patent application EP-A-0 518 773. Commercial pigments that may be mentioned include the products sold by the companies Sachtleben Pigments, Tayca, Merck and Degussa.

The metal oxide pigments may be coated or uncoated.

The coated pigments are pigments that have undergone one or more surface treatments of chemical, electronic, mechanochemical and/or mechanical nature with compounds such as amino acids, beeswax, fatty acids, fatty alcohols, anionic surfactants, lecithins, sodium, potassium, zinc, iron or aluminium salts of fatty acids, metal alkoxides (of titanium or aluminium), polyethylene, silicones, proteins (collagen, elastin), alkanolamines, silicon oxides, metal oxides or sodium hexametaphosphate.

The coated pigments are more particularly titanium oxides that have been coated:

- with silica, such as the product Sunveil from the company Ikeda,
- with silica and iron oxide, such as the product Sunveil F from the company Ikeda,
- with silica and alumina, such as the products Microtitanium Dioxide MT 500 SA and Microtitanium Dioxide MT 100 SA from the company Tayca and Tioveil from the company Tioxide,
- with alumina, such as the products Tipaque TTO-55 (B) and Tipaque TTO-55 (A) from the company Ishihara and UVT 14/4 from the company Sachtleben Pigments,
- with alumina and aluminium stearate, such as the products Microtitanium Dioxide MT 100 T, MT 100 TX, MT 100 Z and MT-01 from the company Tayca, the products Solaveil CT-10 W and Solaveil CT 100 from the company Uniqema and the product Eusolex T-AVO from the company Merck,
- with silica, alumina and alginic acid, such as the product MT-100 AQ from the company Tayca,
- with alumina and aluminium laurate, such as the product Microtitanium Dioxide MT 100 S from the company Tayca,
- with iron oxide and iron stearate, such as the product Microtitanium Dioxide MT 100 F from the company Tayca,
- with zinc oxide and zinc stearate, such as the product BR 351 from the company Tayca,
- with silica and alumina and treated with a silicone, such as the products Microtitanium Dioxide MT 600 SAS, Microtitanium Dioxide MT 500 SAS or Microtitanium Dioxide MT 100 SAS from the company Tayca,
- with silica, alumina and aluminium stearate and treated with a silicone, such as the product STT-30-DS from the company Titan Kogyo,
- with silica and treated with a silicone, such as the product UV-Titan X 195 from the company Sachtleben Pigments,
- with alumina and treated with a silicone, such as the products Tipaque TTO-55 (S) from the company Ishihara or UV Titan M 262 from the company Sachtleben Pigments,
- with triethanolamine, such as the product STT-65-S from the company Titan Kogyo,
- with stearic acid, such as the product Tipaque TTO-55 (C) from the company Ishihara,
- with sodium hexametaphosphate, such as the product Microtitanium Dioxide MT 150 W from the company Tayca,
- $TiO_2$ treated with octyltrimethylsilane, sold under the trade name T 805 by the company Degussa Silices,
- $TiO_2$ treated with a polydimethylsiloxane, sold under the trade name 70250 Cardre UF $TiO_2SI_3$ by the company Cardre,
- anatase/rutile $TiO_2$ treated with a polydimethylhydrosiloxane, sold under the trade name Microtitanium Dioxide USP Grade Hydrophobic by the company Color Techniques.

Mention may also be made of $TiO_2$ pigments doped with at least one transition metal such as iron, zinc or manganese and more particularly manganese. Preferably, the said doped pigments are in the form of an oily dispersion. The oil present in the oily dispersion is preferably chosen from triglycerides including those of capric/caprylic acids. The oily dispersion of titanium oxide particles may also comprise one or more dispersants, for instance a sorbitan ester, for instance sorbitan isostearate, or a polyoxyalkylenated fatty acid ester of glycerol, for instance TRI-PPG-3 myristyl ether citrate and polyglyceryl-3 polyricinoleate. Preferably, the oily dispersion of titanium oxide particles comprises at least one dispersant chosen from polyoxyalkylenated fatty acid esters of glycerol. Mention may be made more particularly of the oily dispersion of $TiO_2$ particles doped with manganese in capric/caprylic acid triglyceride in the presence of TRI-PPG-3 myristyl ether citrate and polyglyceryl-3 polyricinoleate and sorbitan isostearate having the INCI name: Titanium dioxide (and) TRI-PPG-3 myristyl ether citrate (and) polyglyceryl-3 ricinoleate (and) sorbitan isostearate, for instance the product sold under the trade name Optisol TD50 by the company Croda.

The uncoated titanium oxide pigments are sold, for example, by the company Tayca under the trade names Microtitanium Dioxide MT 500 B or Microtitanium Dioxide MT 600 B, by the company Degussa under the name P 25, by the company Wackher under the name Transparent titanium oxide PW, by the company Miyoshi Kasei under the name UFTR, by the company Tomen under the name ITS and by the company Tioxide under the name Tioveil AQ.

The uncoated zinc oxide pigments are, for example:

- those sold under the name Z-Cote by the company Sunsmart;
- those sold under the name Nanox by the company Elementis;
- those sold under the name Nanogard WCD 2025 by the company Nanophase Technologies.

The coated zinc oxide pigments are for example:

- those sold under the name Zinc Oxide CS-5 by the company Toshibi (ZnO coated with polymethylhydrogenosiloxane);
- those sold under the name Nanogard Zinc Oxide FN by the company Nanophase Technologies (as a 40% dispersion in Finsolv TN, $C_{12}$-$C_{15}$ alkyl benzoate);
- those sold under the name Daitopersion Zn-30 and Daitopersion Zn-50 by the company Daito (dispersions in cyclopolymethylsiloxane/oxyethylenated polydimethylsiloxane, containing 30% or 50% of zinc oxides coated with silica and polymethylhydrogenosiloxane);
- those sold under the name NFD Ultrafine ZnO by the company Daikin (ZnO coated with perfluoroalkyl phosphate and copolymer based on perfluoroalkylethyl as a dispersion in cyclopentasiloxane);
- those sold under the name SPD-Z1 by the company Shin-Etsu (ZnO coated with silicone-grafted acrylic polymer, dispersed in cyclodimethylsiloxane);
- those sold under the name Escalol Z100 by the company ISP (alumina-treated ZnO dispersed in an ethylhexyl methoxycinnamate/PVP-hexadecene copolymer/methicone mixture);
- those sold under the name Fuji ZnO-SMS-10 by the company Fuji Pigment (ZnO coated with silica and polymethylsilsesquioxane);
- those sold under the name Nanox Gel TN by the company Elementis (ZnO dispersed at a concentration of 55% in $C_{12}$-$C_{15}$ alkyl benzoate with hydroxystearic acid polycondensate).

The uncoated cerium oxide pigments may be, for example, those sold under the name Colloidal Cerium Oxide by the company Rhone-Poulenc.
The uncoated iron oxide pigments are, for example, sold by the company Arnaud under the names Nanogard WCD 2002 (FE 45B), Nanogard Iron FE 45 BL AQ, Nanogard FE 45R AQ and Nanogard WCD 2006 (FE 45R) or by the company Mitsubishi under the name TY-220.
The coated iron oxide pigments are sold, for example, by the company Arnaud under the names Nanogard WCD 2008 (FE 45B FN), Nanogard WCD 2009 (FE 45B 556), Nanogard FE 45 BL 345 and Nanogard FE 45 BL or by the company BASF under the name Transparent Iron Oxide.
Mention may also be made of mixtures of metal oxides, in particular of titanium dioxide and of cerium dioxide, including the equal-weight mixture of titanium dioxide and cerium dioxide coated with silica, sold by the company Ikeda under the name Sunveil A, and also the mixture of titanium dioxide and zinc dioxide coated with alumina, silica and silicone, such as the product M 261 sold by the company Sachtleben Pigments, or coated with alumina, silica and glycerol, such as the product M 211 sold by the company Sachtleben Pigments.
According to the invention, coated or uncoated titanium oxide pigments are particularly preferred.
The additional UV-screening agents according to the invention are preferably present in the compositions according to the invention in a content ranging from 0.1% to 45% by weight and in particular from 5% to 30% by weight relative to the total weight of the composition.

**b) Other additives:**

The compositions in accordance with the present invention may also comprise conventional cosmetic adjuvants chosen in particular from organic solvents, ionic or nonionic thickeners, softeners, humectants, opacifiers, stabilizers, emollients, silicones, antifoams, fragrances, preserving agents, anionic, cationic, nonionic, zwitterionic or amphoteric surfactants, active agents, fillers, polymers, propellants, basifying or acidifying agents or any other ingredient commonly used in the cosmetic and/or dermatological field.
Mention may be made, among organic solvents, of alcohols other than $C_1$-$C_4$ monoalkanols as defined above and in particular short-chain $C_2$-$C_8$ polyols, such as glycerol or diols, such as caprylyl glycol, 1,2-pentanediol, propanediol, butanediol, glycols and glycol ethers, such as ethylene glycol, propylene glycol, butylene glycol, dipropylene glycol or diethylene glycol.
Mention may be made, as thickeners, of carboxyvinyl polymers, such as the Carbopols® (Carbomers) and the Pemulens, such as Pemulen TR1® and Pemulen TR2® (acrylate/$C_{10}$-$C_{30}$ alkyl acrylate copolymer); polyacrylamides, for instance the crosslinked copolymers sold under the names Sepigel 305® (CTFA name: polyacrylamide/$C_{13-14}$ iso-

paraffin/laureth 7) or Simulgel 600 (CTFA name: acrylamide/sodium acryloyldimethyl taurate copolymer/isohexadecane/polysorbate 80) by the company SEPPIC; 2-acrylamido-2-methylpropanesulfonic acid polymers and copolymers, optionally crosslinked and/or neutralized, such as the poly(2-acrylamido-2-methylpropanesulfonic acid) sold by the company Hoechst under the trade name Hostacerin AMPS® (CTFA name: ammonium polyacryloyldimethyl taurate) or Simulgel 800®, sold by the company SEPPIC (CTFA name: sodium polyacryloyldimethyl taurate/polysorbate 80/sorbitan oleate); copolymers of 2-acrylamido-2-methylpropanesulfonic acid and of hydroxyethyl acrylate, such as Simulgel NS® and Sepinov EMT 10®, sold by the company SEPPIC; cellulose derivatives, such as hydroxyethylcellulose; polysaccharides and in particular gums, such as xanthan gum; water-soluble or water-dispersible silicone derivatives, such as acrylic silicones, polyether silicones and cationic silicones, and mixtures thereof.

Among the acidifying agents, examples that may be mentioned include mineral or organic acids, for instance hydrochloric acid, orthophosphoric acid, sulfuric acid, carboxylic acids, for instance acetic acid, tartaric acid, citric acid or lactic acid, and sulfonic acids.

Among the basifying agents, examples that may be mentioned include aqueous ammonia, alkali metal carbonates, alkanolamines, such as mono-, di- and triethanolamines and derivatives thereof, sodium hydroxide or potassium hydroxide.

Preferably, the cosmetic composition comprises one or more basifying agents selected from alkanolamines, in particular triethanolamine, and sodium hydroxide.

In the case of a direct emulsion, the pH of the composition in accordance with the invention is generally between 3 and 12 approximately, preferably between 5 and 11 approximately and more particularly still from 6 to 8.5.

Among the active agents for caring for keratin materials such as the skin, the lips, the scalp, the hair, the eyelashes or the nails, examples that may be mentioned include:

- vitamins and derivatives or precursors thereof, alone or as mixtures;
- antioxidants;
- free-radical scavengers;
- antipollution agents;
- self-tanning agents;
- antiglycation agents;
- calmatives;
- deodorants;
- essential oils;
- NO-synthase inhibitors;
- agents for stimulating the synthesis of dermal or epidermal macromolecules and/or for preventing their degradation;
- agents for stimulating fibroblast proliferation;
- agents for stimulating keratinocyte proliferation;
- muscle relaxants,
- refreshing agents;
- tensioning agents;
- mattifying agents;
- depigmenting agents;
- propigmenting agents;
- keratolytic agents;
- desquamating agents;
- moisturizers;
- antiinflammatory agents;
- antimicrobial agents;
- slimming agents;
- agents acting on the energy metabolism of cells;
- insect repellents;
- substance P or CRGP antagonists;
- hair-loss counteractants;
- antiwrinkle agents;
- antiageing agents.

A person skilled in the art will select the said active agent(s) as a function of the effect desired on the skin, the hair, the eyelashes, the eyebrows and the nails.

Needless to say, a person skilled in the art will take care to choose the abovementioned optional additional compound or compounds and/or the amounts thereof so that the advantageous properties intrinsically attached to the

compositions in accordance with the invention are not, or not substantially, detrimentally affected by the envisaged addition(s).

## GALENICAL FORMS

The compositions according to the invention may be prepared according to the techniques that are well known to those skilled in the art. They may in particular be in the form of a simple or complex emulsion (O/W, W/O, O/W/O or W/O/W), such as a cream, a milk or a cream gel.

They may also be in anhydrous form, for instance in the form of an oil. The term "anhydrous composition" means a composition containing less than 1% by weight of water, or even less than 0.5% of water, and especially free of water, the water not being added during the preparation of the composition but corresponding to the residual water provided by the mixed ingredients. They may optionally be packaged in aerosol form and may be in the form of a mousse or a spray.

In the case of compositions in the form of oil-in-water or water-in-oil emulsions, the emulsification processes that may be used are of the paddle or impeller, rotor-stator and HPH type.

In order to obtain stable emulsions with a low content of polymer (oil/polymer ratio > 25), it is possible to prepare the dispersion in concentrated phase and then to dilute the dispersion with the remainder of the aqueous phase.

It is also possible, by means of an HPH (between 50 and 800 bar), to obtain stable dispersions with drop sizes that may be as low as 100 nm.

The emulsions generally comprise at least one emulsifier chosen from amphoteric, anionic, cationic and nonionic emulsifiers, used alone or as a mixture. The emulsifiers are appropriately chosen according to the emulsion to be obtained (W/O or O/W).

The compositions according to the invention are preferably in the form of an oil-in-water or water-in-oil emulsion. The emulsifying surfactants are appropriately chosen according to the emulsion to be obtained.

Non-limiting examples of W/O emulsifying surfactants suitable for water-in-oil emulsions are given in particular in the publication entitled McCutcheon's Emulsifiers & Detergents, 1998, International Edition, MC Publishing Company, in the chapter entitled HLB Index.

Examples of W/O emulsifying surfactants that may be mentioned include alkyl esters or ethers of sorbitan, of glycerol, of polyol or of sugars; silicone surfactants, for instance dimethicone copolyols, such as the mixture of cyclomethicone and of dimethicone copolyol, sold under the name DC 5225 C® by the company Dow Corning, and alkyldimethicone copolyols such as laurylmethicone copolyol sold under the name Dow Corning 5200 Formulation Aid by the company Dow Corning; cetyldimethicone copolyol, such as the product sold under the name Abil EM 90R® by the company Goldschmidt, and the mixture of cetyldimethicone copolyol, of polyglyceryl isostearate (4 mol) and of hexyl laurate, sold under the name Abil WE 09® by the company Goldschmidt. It is also possible to add thereto one or more coemulsifiers, which may advantageously be chosen from the group consisting of polyol alkyl esters.

Mention may also be made of non-silicone emulsifying surfactants, in particular alkyl esters or ethers of sorbitan, of glycerol, of polyol or of sugars.

Polyol alkyl esters that may especially be mentioned include polyethylene glycol esters, for instance PEG-30 dipolyhydroxystearate, such as the product sold under the name Arlacel P135® by the company ICI.

Examples of glycerol and/or sorbitan esters that may be mentioned include polyglyceryl isostearate, such as the product sold under the name Isolan GI 34® by the company Goldschmidt; sorbitan isostearate, such as the product sold under the name Arlacel 987® by the company ICI; sorbitan glyceryl isostearate, such as the product sold under the name Arlacel 986® by the company ICI, and mixtures thereof.

For the O/W emulsions, examples of nonionic emulsifying surfactants that may be mentioned include polyoxyalkylenated (more particularly polyoxyethylenated and/or polyoxypropylenated) esters of fatty acids and of glycerol; oxyalkylenated esters of fatty acids and of sorbitan; polyoxyalkylenated (in particular polyoxyethylenated and/or polyoxypropylenated) esters of fatty acids, optionally in combination with an ester of fatty acid and of glycerol, such as the PEG-100 Stearate/Glyceryl Stearate mixture sold, for example, by the company ICI under the name Arlacel 165; oxyalkylenated (oxyethylenated and/or oxypropylenated) ethers of fatty alcohols; esters of sugars, such as sucrose stearate; or ethers of fatty alcohol and of sugar, in particular alkyl polyglucosides (APGs), such as decyl glucoside and lauryl glucoside, sold, for example, by the company Henkel under the respective names Plantaren 2000® and Plantaren 1200®, cetostearyl glucoside, optionally as a mixture with cetostearyl alcohol, sold, for example, under the name Montanov 68® by the company SEPPIC, under the name Tegocare CG90® by the company Goldschmidt and under the name Emulgade KE3302® by the company Henkel, and arachidyl glucoside, for example in the form of the mixture of arachidyl and behenyl alcohols and of arachidyl glucoside sold under the name Montanov 202® by the company SEPPIC. According to a particular embodiment of the invention, the mixture of the alkyl polyglucoside as defined above with the corresponding fatty alcohol may be in the form of a self-emulsifying composition, for example as described in document WO-A-92/06778.

According to a particularly preferred form, the compositions are in the oil-in-water form.

When it is an emulsion, the aqueous phase of this emulsion may comprise a nonionic vesicular dispersion prepared according to known processes (Bangham, Standish and Watkins, J. Mol. Biol., 13, 238 (1965), FR 2 315 991 and FR 2 416 008).

The compositions according to the invention find their application in a large number of treatments, especially cosmetic treatments, of the skin, the lips and the hair, including the scalp, especially for protecting and/or caring for the skin, the lips and/or the hair, and/or for making up the skin and/or the lips.

Another subject of the present invention consists of the use of the compositions according to the invention as defined above for the manufacture of products for cosmetically treating the skin, the lips, the nails, the hair, the eyelashes, the eyebrows and/or the scalp, especially care products, antisun products and makeup products.

The cosmetic compositions according to the invention may be used, for example, as makeup products.

Another subject of the present invention consists of a non-therapeutic cosmetic process for caring for and/or making up a keratin material, which consists in applying to the surface of the said keratin material at least one composition according to the invention as defined above.

The cosmetic compositions according to the invention may be used, for example, as care products and/or antisun products for the face and/or the body with a liquid to semi-liquid consistency, such as milks, more or less smooth creams, cream gels or pastes. They may optionally be packaged in aerosol form and may be in the form of a mousse or a spray.

The compositions according to the invention in the form of vaporizable fluid lotions in accordance with the invention are applied to the skin or the hair in the form of fine particles by means of pressurization devices. The devices in accordance with the invention are well known to those skilled in the art and comprise non-aerosol pumps or "atomizers", aerosol containers comprising a propellant and aerosol pumps using compressed air as propellant. These devices are described in patents US 4 077 441 and US 4 850 517.

The compositions packaged in aerosol form in accordance with the invention generally contain conventional propellants, for instance hydrofluoro compounds, dichlorodifluoromethane, difluoroethane, dimethyl ether, isobutane, n-butane, propane or trichlorofluoromethane. They are preferably present in amounts ranging from 15% to 50% by weight relative to the total weight of the composition.

## ASSEMBLY

According to another aspect, the invention also relates to a cosmetic assembly comprising:

i) a container delimiting one or more compartment(s), the said container being closed by a closing member and optionally not being leaktight; and
ii) a makeup and/or care composition in accordance with the invention placed inside the said compartment(s).

The container may be, for example, in the form of a jar or a box.

The closing member may be in the form of a lid comprising a cap mounted so as to be able to move by translation or by pivoting relative to the container housing the said makeup and/or care composition(s).

The examples that follow serve to illustrate the invention without, however, being limiting in nature. In these examples, the amounts of the composition ingredients are given as weight percentages relative to the total weight of the composition.

## Example A1: Preparation of compound (1)

(1) 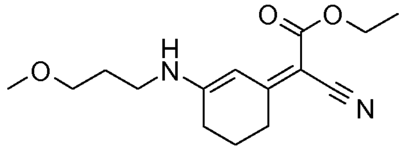

122.23 g of 3-[(3-methoxypropyl)amino]-2-cyclohexen-1-one were alkylated with dimethyl sulfate or alternatively with diethyl sulfate and treated with 75.45 g of ethyl cyanoacetate in approximately equimolar proportions in the presence of a base and optionally of a solvent.

The following base/solvent combinations were used:

| Example | Base | Solvent |
|---|---|---|
| Example A1.1 | DBU (1,8-diazabicyclo[5.4.0]undec-7-ene) | dimethylacetamide |
| Example A1.2 | triethylamine | isopropanol |
| Example A1.3 | 3-methoxypropylamine | isopropanol |
| Example A1.4 | 3-methoxypropylamine | tert-amyl alcohol |
| Example A1.5 | 3-methoxypropylamine | toluene |
| Example A1.6 | 3-methoxypropylamine | dimethylformamide |
| Example A1.7 | 3-methoxypropylamine | no solvent |
| Example A1.8 | N-morpholine | isopropanol |

The completion of the alkylation reaction was monitored, for example, via methods such as TLC, GC or HPLC.
162.30 g of compound (14) were obtained in the form of a brown oil.
After crystallization, the product was obtained in the form of yellowish crystals.
Melting point: 92.7°C

**Example A2: Preparation of compound (2)**

(2)

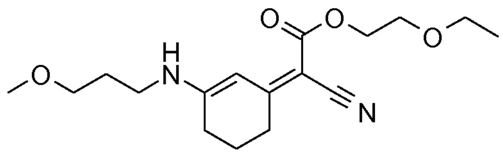

148.4 g of 3-[(3-methoxypropyl)amino]-2-cyclohexen-1-one were alkylated with dimethyl sulfate or alternatively with diethyl sulfate and treated with 130.00 g of 2-ethoxyethyl cyanoacetate in the presence of an organic base and a solvent.
The following base/solvent combinations were used:

| Example | Base | Solvent |
|---|---|---|
| Example A2.1 | DBU (1,8-diazabicyclo[5.4.0]undec-7-ene) | dimethylacetamide |
| Example A2.2 | triethylamine | isopropanol |
| Example A2.3 | 3-methoxypropylamine | isopropanol |
| Example A2.4 | N-methylmorpholine | tert-amyl alcohol |
| Example A2.5 | 3-methoxypropylamine | toluene |
| Example A2.6 | 3-methoxypropylamine | dimethylformamide |
| Example A2.7 | 3-methoxypropylamine | No solvent |

**Example A3 (outside the invention): Preparation of the compound (2Z)-2-cyano-N-(3-methoxypropyl)-2-{3-[(3-methoxypropyl)amino]cyclohex-2-en-1-ylidene}ethanamide described in the unpublished patent application PCT/EP 2012/064 195**

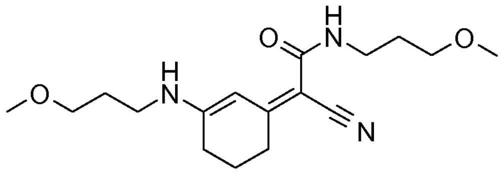

101.00 g of 3-[(3-methoxypropyl)amino]-2-cyclohexen-1-one were alkylated with dimethyl sulfate or alternatively with diethyl sulfate and treated with 86.00 g of 2-cyano-N-(3-methoxypropyl)acetamide in approximately equimolar proportions in the presence of a base and optionally of a solvent.

The following base/solvent combinations were used:

| Example | Base | Solvent |
|---|---|---|
| Example A3.1 | DBU (1,8-diazabicyclo[5.4.0]undec-7-ene) | dimethylacetamide |
| Example A3.2 | triethylamine | isopropanol |
| Example A3.3 | 3-methoxypropylamine | isopropanol |
| Example A3.4 | 3-methoxypropylamine | tert-amyl alcohol |
| Example A3.5 | 3-methoxypropylamine | toluene |
| Example A3.6 | 3-methoxypropylamine | dimethylformamide |
| Example A3.7 | 3-methoxypropylamine | no solvent |

The crude product (2Z)-2-cyano-N-(3-methoxypropyl)-2-{3-[(3-methoxypropyl)amino]cyclohex-2-en-1-ylidene}ethanamide was obtained in the form of a dark brown oil.

After chromatography on a column of silica gel (eluent: 99/1 toluene/methanol), 81.8 g of product were obtained in the form of yellowish crystals.
Melting point: 84.7-85.3°C.

**Formulation Examples 1 to 8**

The compound 2-ethoxyethyl (2Z)-cyano{3-[(3-methoxypropyl)amino]cyclohex-2-en-1-ylidene}ethanoate (2) of the invention was compared with:

- the compound (2Z)-2-cyano-N-(3-methoxypropyl)-2-{3-[(3-methoxypropyl)amino]cyclohex-2-en-1-ylidene}ethanamide according to Example A3 (outside the invention) ;
- the compound octyl-5-N,N-diethylamino-2-phenylsulfonyl-2,4-pentadienoate (outside the invention) ;
- the merocyanine compound MC11 disclosed in the application WO2008/090066 (outside the invention).

Formulations 1 to 3 below were prepared; they were constructed such that the sum of the contents of oil and of liposoluble UV-screening agents remains constant. The content of the screening agents was adjusted so as to ensure the same level of UVB screening and also the same *in vitro* SPF, and also the same absorbance profile between 290 and 340 nm. For each of the formulations, the SPF, the $UVA_{PPD}$ index and the absorbance after 24 hours at room temperature were measured. The amounts are expressed as weight percentages relative to the total weight of the composition.

| Phase | Ingredients | Formulation 1 (outside the invention) | Formulation 2 (outside the invention) | Formulation 3 (invention) |
|---|---|---|---|---|
| A | Water | qs 100 | qs 100 | qs 100 |
| | Glycerin | 6 | 6 | 6 |
| | Disodium EDTA | 0.1 | 0.1 | 0.1 |
| | Triethanolamine | 0.45 | 0.45 | 0.45 |
| | Potassium cetyl phosphate (Amphisol K®) | 1 | 1 | 1 |
| B | 2-Ethylphenyl benzoate (X-Tend226®) | 24.4 | 23.7 | 23.4 |
| | n-Hexyl 2-(4-diethylamino-2-hydroxybenzoyl)benzo ate (Uvinul A + ®) | 4 | 4 | 4.3 |
| | Octyl 5-N,N-diethylamino-2-phenylsulfonyl-2,4-pentad ienoate | 1.6 | - | - |
| | (2Z)-2-cyano-N-(3-methoxypropyl)-2-{3-[(3-methoxypropyl)amino]-cyclohex-2-en-1-ylidene}ethanamide | - | 2.3 | - |
| | Compound (2) | - | - | 2.3 |
| | Stearic acid | 1.5 | 1.5 | 1.5 |
| | Glyceryl stearate (and) PEG-100 stearate (Arlacel 165) | 1.5 | 1.5 | 1.5 |
| | Dimethicone | 0.5 | 0.5 | 0.5 |
| | Preservatives | 1.28 | 1.28 | 1.28 |
| C | Isohexadecane | 2 | 2 | 2 |
| | Xanthan gum | 0.1 | 0.1 | 0.1 |
| | Acrylates/C10-30 alkyl acrylate crosspolymer (Pemulen TR1®) | 0.25 | 0.25 | 0.25 |
| D | Triethanolamine | 0.25 | 0.25 | 0.25 |
| E | Alcohol | 2 | 2 | 2 |
| **in vitro SPF ($t_{24h}$)** | 2.4 ± 0.2 | 2.4 ± 0.1 | 2.4 ± 0.1 | |
| **in vitro UVAPPD ($t_{24h}$)** | 11.0 ± 1.0 | 11.5 ± 2.0 | 11.6 ± 2.0 | |

Formulations 4 to 8 below were prepared. The content of the filters was constant in order to compare the performance of the compound (2) according to the invention to the one of the compound (2Z)-2-cyano-N-(3-methoxypropyl)-2-{3-[(3-methoxypropyl)amino]cyclohex-2-en-1-ylidene}-ethanamide according to example A3 (outside the invention) and to the one of the compound MC11 disclosed in the application WO2008/090066 (outside the invention) at the same content. For some of these formulations, the SPF, the $UVA_{PPD}$ index and the absorbance 24 hours after formulation were measured. The amounts are expressed as weight percentages relative to the total weight of the composition.

| Phase | Ingredients | Formulation 4 (outside the invention) | Formulation 5 (invention) |
|---|---|---|---|
| A | Water | qsp 100 | qsp 100 |
| | Glycerin | 5 | 5 |
| | Disodium EDTA | 0.1 | 0.1 |
| | Triethanolamine | 0.45 | 0.45 |
| | Potassium Cetyl Phosphate(Amphisol K®) | 1 | 1 |
| B | Isopropyl Lauroyl Sarcosinate (Eldew SL-205®) | 30 | 30 |
| | (2Z)-2-cyano-N-(3-methoxypropyl)-2-{3-[(3-methoxypropyl)amino]cyclohe x-2-en-1-ylidene} ethanamide | 2 | - |
| | Compound (2) | - | 2 |
| | n-hexyl 2-(4-diethylamino-2-hydroxybenzoyl)benzoate (Uvinul A + ®) | 3 | 3 |
| | Stearic Acid | 1.5 | 1.5 |
| | Glyceryl Stearate (and) PEG-100 Stearate (Arlacel 165®) | 2.5 | 2.5 |
| | Dimethicone | 0.5 | 0.5 |
| | Cetyl Alcohol | 0.5 | 0.5 |
| | Cetearyl Alcohol (and) Cetearyl Glucoside (Montanov 68®) | 2 | 2 |
| | Preservatives | 1 | 1 |
| C | Isohexadecane | 1 | 1 |
| | Xanthan Gum | 0.2 | 0.2 |
| | Acrylates / C10-C30 Alkyl Acrylate crosspolymer (Pemulen TR1®) | 0.2 | 0.2 |
| D | Triethanolamine | 0.2 | 0.2 |
| **SPF in vitro ($t_{24h}$)** | | 2.3 ± 0,1 | 2.3 ± 0.1 |
| **UVA PPD in vitro ($t_{24h}$)** | | 23.6 ± 7.1 | 22.8 ± 1.6 |

| Phase | Ingredients | Formulation 5 (invention) | Formulation 6 (outside the invention) | Formulation 7 (outside the invention) | Formulation 8 (outside the invention) |
|---|---|---|---|---|---|
| A | Water | qsp 100 | qsp 100 | qsp 100 | qsp 100 |
| | Glycerin | 5 | 5 | 5 | 5 |
| | Disodium EDTA | 0,1 | 0,1 | 0,1 | 0,1 |
| | Triethanolamine | 0,45 | 0,45 | 0,45 | 0,45 |
| | Potassium Cetyl Phosphate(Amphisol K®) | 1 | 1 | 1 | 1 |

(continued)

| Phase | Ingredients | Formulation 5 (invention) | Formulation 6 (outside the invention) | Formulation 7 (outside the invention) | Formulation 8 (outside the invention) |
|---|---|---|---|---|---|
| B | Isopropyl Lauroyl Sarcosinate (Eldew SL-205®) | 30 | 30 | 30 | 30 |
| | Compound (2) | 2 | - | 2 | - |
| | MC11 of WO2008/090066 | - | 2 | - | 2 |
| | n-hexyl 2-(4-diethylamino-2-hydroxybenzoyl) benzoat e (Uvinul A + ®) | 3 | 3 | - | - |
| | Stearic Acid | 1,5 | 1,5 | 1,5 | 1,5 |
| | Glyceryl Stearate (and) PEG-100 Stearate (Arlacel 165®) | 2,5 | 2,5 | 2,5 | 2,5 |
| | Dimethicone | 0,5 | 0,5 | 0,5 | 0,5 |
| | Cetyl Alcohol | 0,5 | 0,5 | 0,5 | 0,5 |
| | Cetearyl Alcohol (and) Cetearyl Glucoside (Montanov 68®) | 2 | 2 | 2 | 2 |
| | Preservatives | 1 | 1 | 1 | 1 |
| C | Isohexadecane | 1 | 1 | 1 | 1 |
| | Xanthan Gum | 0,2 | 0,2 | 0,2 | 0,2 |
| | Acrylates / C10-C30 Alkyl Acrylate crosspolymer (Pemulen TR1®) | 0,2 | 0,2 | 0,2 | 0,2 |
| D | Triethanolamine | 0,2 | 0,2 | 0,2 | 0,2 |

**Emulsion preparation method:**

The aqueous phase A and oily phase B were prepared by mixing the starting materials with mechanical stirring at 80°C. Once the aqueous solution A and oily solution B were macroscopically homogeneous, the emulsion was prepared by introducing phase B into phase A with stirring using a rotor-stator homogenizer with a stirring speed of 4500 rpm for 20 minutes. Phases C and then D were then successively added, with continued stirring. The emulsion was finally cooled to room temperature before adding phase E, when it exists. The final emulsion was characterized by drops between 1 μm and 20 μm in size.

**In vitro protocol for evaluating the screening efficacy**

The sun protection factor (SPF) was determined according to the in vitro method described by B.L. Diffey in J. Soc. Cosmet. Chem. 40, 127-133 (1989). The measurements were carried out using a UV-1000S spectrophotometer from the company Labsphere. The "static in vitro protection factor (SPF)" value is extracted. Each composition is applied to a rough plate of PMMA in the form of a uniform and even deposit in a proportion of 1 mg/cm$^2$.

The in vitro UVAPPD index measurements were taken under the same conditions using a UV-1000S spectrophotometer from the company Labsphere. The "UV-APPD index (persistent pigment darkening action spectrum)" value is extracted. Each composition was applied to a rough plate of PMMA, in the form of a homogeneous and even deposit at a rate of 1 mg/cm$^2$.

**Protocol for evaluating the absorbance spectra of the formulations**

The absorbance spectra of the formulations were extracted from the mAF data as a function of the wavelength generated during the in vitro SPF measurement and the in vitro UVAPPD index measurement 24 hours after the formulation. The mAF values were then converted into absorbance values according to: Abs= log(mAF).

**Absorbance of the formulations measured 24 hours after formulation at room temperature**

| Absorbance | Formulation 1 (outside the invention) | Formulation 2 (outside the invention) | Formulation 3 (invention) |
|---|---|---|---|
| Absorbance at 290 nm after 24 hours | 0.36 ± 0.04 | 0.32 ± 0.03 | 0.33 ± 0.02 |
| Absorbance at 320 nm after 24 hours | 0.49 ± 0.03 | 0.48 ± 0.03 | 0.48 ± 0.03 |
| Absorbance at 400 nm after 24 hours | 0.27 ± 0.02 | 0.70 ± 0.04 | 0.80 ± 0.04 |

| Absorbance | Formulation 4 (outside the invention) | Formulation 5 (invention) |
|---|---|---|
| Absorbance at 290 nm after 24 hours | 0.21 ± 0.02 | 0.20 ± 0.01 |
| Absorbance at 320 nm after 24 hours | 0.54 ± 0.02 | 0.51 ± 0.01 |
| Absorbance at 400 nm after 24 hours | 0.71 ± 0.01 | 0.87 ± 0.01 |

The absorbance values at 400 nm measured 24 hours after formulation showed that for a same SPF in vitro, same UVA PPD index in vitro, the formulations 1 and 2 (outside the invention) are less efficient than the formulation 3 according to the invention and that the formulation 4 ((outside the invention) is less efficient than the formulation 5 according to the l'invention in view of the filtration of UVA radiations.

**Protocol for evaluating the color of the formulations**

The color of the formulations was evaluated after preparation of thin films on contrast map. The formulations were deposited within a circle of 2.2 cm of diameter and planed to obtain thicknesses of reproducible deposit. The colorimetric measures were then made by means of a spectro-colorimeter Minolta CM2600D in two points of the film. This operation is twice reproduced, which leads to 4 experimental values by formulation.

The results are expressed in the system (L *, has *, b *) in which L* represents the luminance, a* represents the red-green axis (-a* = green, +a* = red) and b* represents the yellow-blue axis (-b* blue, +b* yellow). So, a* and b* express the shade of the compound.

The difference of color ΔE* was calculated from the variations ΔL*, Δa* et Δb* between the compound (2) and the compound MC11 with the following equation :

$$(\Delta E^*)^2 = (\Delta L^*)^2 + (\Delta a^*)^2 + (\Delta b^*)^2$$

ΔL* = L* formulation with compound MC11 - L* formulation with compound (2)
Δa* = a* formulation with compound MC11 - a* formulation with compound (2)
Δb* = b* formulation with compound MC11 - b* formulation with compound (2)

We consider that the difference of color between the two compounds is significant if ΔE* > 2.

**Colorimetric measures on the formulations 5 to 8**

| | Formulation 5 (invention) | Formulation 6 (outside the invention) | Formulation 7 (outside the invention) | Formulation 8 (outside the invention) |
|---|---|---|---|---|
| L* | 93.3 ± 0.6 | 92.8 ± 0.7 | 93.1 ± 0.6 | 93.0 ± 0.6 |
| a* | -5.8 ± 0.2 | -6.4 ± 0.1 | -5,08 ± 0,03 | -6.40 ± 0.05 |
| Δa* | -0,7 | | -1,32 | |
| b* | 13.7 ± 0.3 | 17.2 ± 0.3 | 12.0 ± 0,2 | 15.8 ± 0.2 |
| **Δb*** | **3.5** | | **3.8** | |
| **ΔE*** | **3.6** | | **4.0** | |

The colorimetry results on the examples 5 to 8 show that the formulation 5 with the compound (2) is significantly less yellow than the equivalent formulations 6,7 and 8 with the compound MC11 of the application WO2008/ 090066.

**Formulation examples 9 to 11**

The compound 2-ethoxyethyl (2Z)-cyano{3-[(3-methoxypropyl)amino]cyclohex-2-en-1-ylidene}ethanoate (2) of the invention was compared with:

- the compound (2Z)-2-cyano-N-(3-methoxypropyl)-2-{3-[(3-methoxypropyl)amino]cyclohex-2-en-1-ylidene}ethanamide according to Example A3 (outside the invention)
- the compound octyl-5-N,N-diethylamino-2-phenylsulfonyl-2,4-pentadienoate (outside the invention)

Formulations 9 to 11 below were prepared; they were constructed such that the sum of the contents of oil and of liposoluble UV-screening agents remains constant. The content of the screening agents was adjusted so as to ensure the same level of UVB screening and also the same *in vitro* SPF, and also the same absorbance profile between 290 and 340 nm. For each of the formulations, the SPF, the $UVA_{PPD}$ index and the absorbance after 24 hours at room temperature were measured. The amounts are expressed as weight percentages relative to the total weight of the composition.

| Phase | Ingredients | Formulation 9 (outside the invention) | Formulation 10 (outside the invention) | Formulation 11 (invention) |
|---|---|---|---|---|
| A | Water | qs 100 | qs 100 | qs 100 |
| | Glycerol | 6 | 6 | 6 |
| | Disodium EDTA | 0.1 | 0.1 | 0.1 |
| | Triethanolamine | qs pH 8.5 | qs pH 8.5 | qs pH 8.5 |
| | Potassium cetyl phosphate (Amphisol K®) | 1 | 1 | 1 |
| | Terephthalylidene dicamphor sulfonic acid (Mexoryl SX® as a percentage of active material) | 5.3 | 5.3 | 5.5 |

(continued)

| Phase | Ingredients | Formulation 9 (outside the invention) | Formulation 10 (outside the invention) | Formulation 11 (invention) |
|---|---|---|---|---|
| B | 2-Ethylphenyl benzoate (X-Tend 226®) | 24.4 | 23.7 | 23.4 |
| | Octyl 5-N,N-diethylamino-2-phenylsulfonyl-2,4-pentad ienoate | 1.4 | - | - |
| | (2Z)-2-cyano-N-(3-methoxypropyl)-2-{3-[(3-methoxypropyl)amino] cyclohex-2-en-1-ylidene}ethanamide | - | 1.9 | - |
| | Compound (2) | - | - | 2.3 |
| | Stearic acid | 1.5 | 1.5 | 1.5 |
| | Glyceryl stearate (and) PEG-100 stearate (Arlacel 165) | 1.5 | 1.5 | 1.5 |
| | Dimethicone | 0.5 | 0.5 | 0.5 |
| | Preserving agents | 1.28 | 1.28 | 1.28 |
| C | Isohexadecane | 2 | 2 | 2 |
| | Xanthan gum | 0.1 | 0.1 | 0.1 |
| | Acrylates/$C_{10}$-$C_{30}$ alkyl acrylate crosspolymer (Pemulen TR1®) | 0.25 | 0.25 | 0.25 |
| D | Triethanolamine | 0.25 | 0.25 | 0.25 |
| E | Alcohol | 2 | 2 | 2 |
| ***in vitro* SPF ($t_{24h}$)**<br>***in vitro* $UVA_{PPD}$ ($t_{24h}$)** | | 4.4 ± 0.4<br>11.4 ± 1.2 | 6.1 ± 1.1<br>14.2 ± 4.0 | 4.9 ± 0.3<br>11.3 ± 0.8 |
| ***in vitro* SPF ($t_{5d}$ at 60°C)**<br>***in vitro* $UVA_{PPD}$ ($t_{5d}$ at 60°C)** | | -<br>- | 4.6 ± 0.1<br>9.0 ± 0.2 | 4.9 ± 0.3<br>11.2 ± 0.9 |

Emulsions 9 to 11 were prepared according to the same preparation mode as for Examples 1 to 8.

The *in vitro* SPF and $UVA_{PPD}$ index values were measured under the same conditions indicated previously.

**Protocol for evaluating the absorbance spectra of the formulations**

The absorbance spectra of the formulations were extracted from the mAF data as a function of the wavelength generated during the *in vitro* SPF measurement and the *in vitro* PPD measurement. The mAF values were then converted into absorbance values according to: Abs= log(mAF).

**Absorbance of the formulations measured after 24 hours**

| Absorbance | Formulation 9 (outside the invention) | Formulation 10 (outside the invention) | Formulation 11 (invention) |
|---|---|---|---|
| Absorbance at 320 nm after 24 hours | 0.70 ± 0.05 | 0.91 ± 0.11 | 0.81 ± 0.03 |
| Absorbance at 360 nm after 24 hours | 1.23 ± 0.05 | 1.19 ± 0.11 | 1.10 ± 0.03 |
| Absorbance at 400 nm after 24 hours | 0.19 ± 0.01 | 0.81 ± 0.03 | 0.82 ± 0.02 |

**Absorbance of the formulations measured after 10 days at 60°C**

| **Absorbance** | **Formulation 10 (outside the invention)** | **Formulation 11 (invention)** |
|---|---|---|
| Absorbance at 320 nm after 10 days at 60°C | 0.78 ± 0.03 | 0.80 ± 0.03 |
| Absorbance at 360 nm after 10 days at 60°C | 0.99 ± 0.01 | 1.10 ± 0.03 |
| Absorbance at 400 nm after 10 days at 60°C | 0.60 ± 0.02 | **0.82 ± 0.02** |

**Conclusions**

The absorbance values at 400 nm measured 24 hours after formulation show that for the same *in vitro* SPF value and the same *in vitro* $UVA_{PPD}$ index, formulation 9 (outside the invention) is less efficient than formulation 11 of the invention in terms of screening out UVA. The absorbance values at 400 nm measured after 10 days of storage at 60°C show that for the same *in vitro* SPF value and the same $UVA_{PPD}$ index, formulation 10 (outside the invention) is less efficient than formulation 11 of the invention in terms of screening out UVA.

**Claims**

1. Cosmetic or dermatological composition comprising, in a physiologically acceptable support:

   a) at least one oily phase and
   b) at least one merocyanine compound corresponding to formula (1) below, and also the E/E- or E/Z- geometrical isomer forms thereof:

   (1) 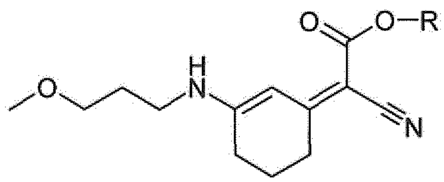

   in which:
   R is a $C_1$-$C_{22}$ alkyl group, a $C_2$-$C_{22}$ alkenyl group, a $C_2$-$C_{22}$ alkynyl group, a $C_3$-$C_{22}$ cycloalkyl group or a $C_3$-$C_{22}$ cycloalkenyl group, the said groups possibly being interrupted with one or more O, and
   c) at least one UVA-screening agent chosen from:

   (i) an amino-substituted 2-hydroxybenzophenone compound and/or
   (ii) a hydrophilic organic UVA-screening agent, and the said hydrophilic organic UVA-screening agent being present in an amount from 0.6% to 15% by weight relative to the total weight of the composition.

2. Composition according to Claim 1, in which the merocyanine compound(s) of formula (1) are chosen from those in which:
   R is a $C_1$-$C_{22}$ alkyl, which may be interrupted with one or more O.

3. Composition according to Claim 1 or 2, in which the merocyanine compound(s) of formula (1) are chosen from the following compounds, and also the E/E- or E/Z-geometrical isomer forms thereof:

| | | | |
|---|---|---|---|
| 1 | 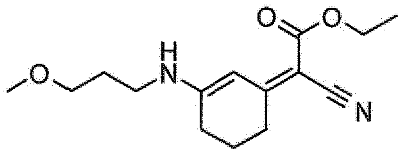<br>ethyl (2Z)-cyano{3-[(3-methoxypropyl)amino] cyclohex-2-en-1-ylidene}ethanoate | 4 | 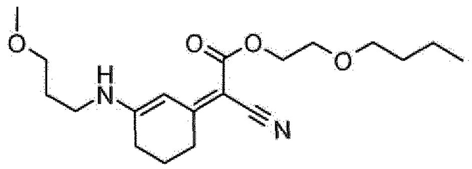<br>2-butoxyethyl (2Z)-cyano{3-[(3-methoxypropyl) amino]cyclohex-2-en-1-ylidene}ethanoate |
| 2 | 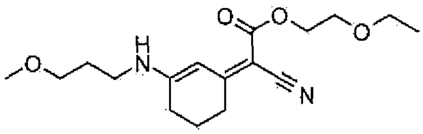<br>2-ethoxyethyl (2Z)-cyano{3-[(3-methoxypropyl) amino]cyclohex-2-en-1-ylidene}ethanoate | 5 | 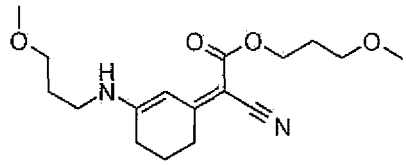<br>3-methoxypropyl (2Z)-cyano{3-[(3-methoxypropyl) amino]cyclohex-2-en-1-ylidene}ethanoate |
| 3 | 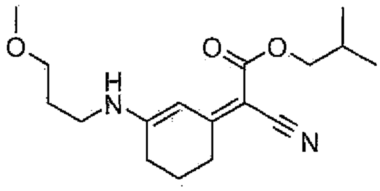<br>2-methylpropyl (2Z)-cyano{3-[(3-methoxypropyl) amino]cyclohex-2-en-1-ylidene}ethanoate | 6 | 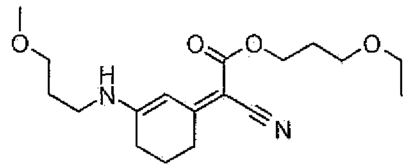<br>3-ethoxypropyl (2Z)-cyano{3-[(3-methoxypropyl) amino]cyclohex-2-en-1-ylidene}ethanoate |

**4.** Composition according to Claim 3, in which the merocyanine compound is 2-ethoxyethyl (2Z)-cyano{3-[(3-methoxypropyl)amino]cyclohex-2-en-1-ylidene}ethanoate (2) in its E/Z geometrical configuration having the following structure:

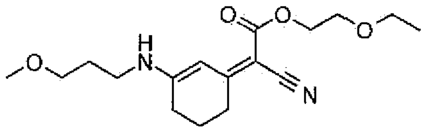

and/or in its E/E geometrical configuration having the following structure:

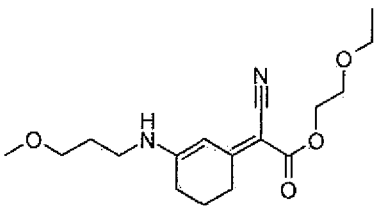

**5.** Composition according to any one of Claims 1 to 4, in which the merocyanine compound(s) of formula (1) are present in a concentration ranging from 0.1 % to 10% by weight and preferentially from 0.2% to 5% by weight relative to the total weight of the composition.

**6.** Composition according to any one of Claims 1 to 5, in which the amino-substituted 2-hydroxybenzophenone compound(s) correspond to formula (I) below:

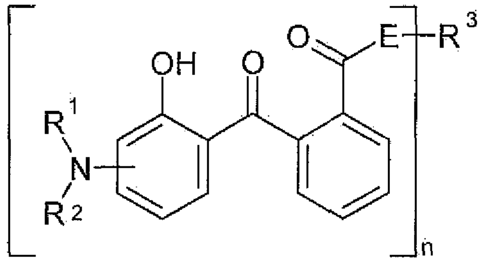

(I)

in which:

$R^1$ and $R^2$, which may be identical or different, denote a $C_1$-$C_{20}$ alkyl radical, a $C_2$-$C_{20}$ alkenyl radical, a $C_3$-$C_{10}$ cycloalkyl radical or a $C_3$-$C_{10}$ cycloalkenyl radical or form, with the nitrogen atom to which they are attached, a 5- or 6-membered ring;
n is a number ranging from 1 to 4;
when n = 1, $R^3$ denotes a $C_1$-$C_{20}$ alkyl radical, a $C_1$-$C_{20}$ alkenyl radical, a $C_1$-$C_5$ hydroxyalkyl radical, a $C_6$-$C_{12}$ cyclohexyl radical, a phenyl which may be substituted with O, N or S, an aminocarbonyl radical or a $C_1$-$C_5$ alkylcarbonyl radical;
when n = 2, $R^3$ denotes an alkyl diradical, a cycloalkyl diradical, an alkenyl diradical or an aryl diradical or $R^3$ with E form a diradical of formula (II):

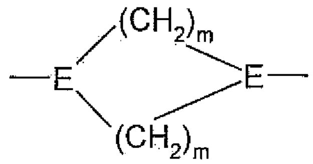

(II)

with m being a number ranging from 1 to 3;
when n = 3, $R^3$ is an alkyl triradical;
when n = 4, $R^3$ is an alkyl tetraradical;
E is -O- or -N($R^4$)- or N;
$R^4$ is hydrogen or a $C_1$-$C_5$ alkyl or $C_1$-$C_5$ hydroxyalkyl radical.

**7.** Composition according to Claim 6, in which the compound of formula (I) is n-hexyl 2-(4-diethylamino-2-hydroxybenzoyl)benzoate of formula (a):

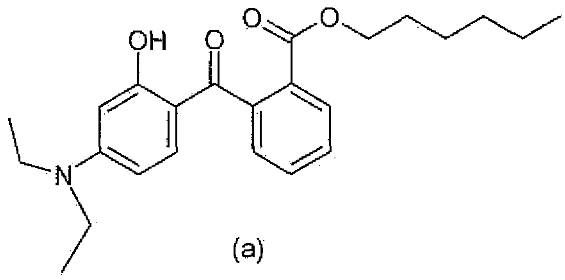

(a)

**8.** Composition according to Claim 6, in which the compound of formula (I) is (2-{4-[2-(4-diethylamino-2-hydroxybenzoyl)benzoyl]piperazine-1-carbonyl}phenyl)(4-diethylamino-2-hydroxyphenyl)methanone of formula (b):

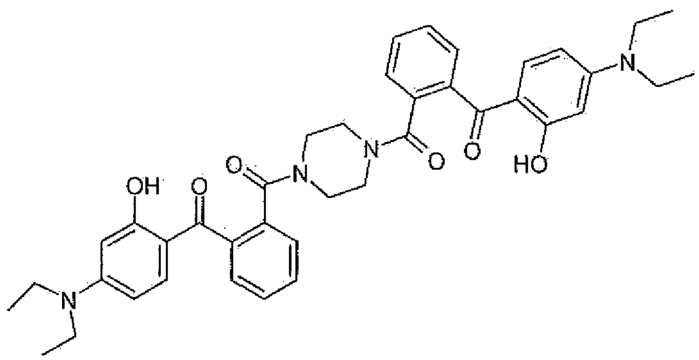

(b)

**9.** Composition according to any one of Claims 1 to 5, in which the hydrophilic organic UVA-screening agent is benzene-1,4-bis(3-methylidene-10-camphorsulfonic acid) or one of the salts thereof, and also the geometrical isomers thereof, corresponding to the general formula (I) below:

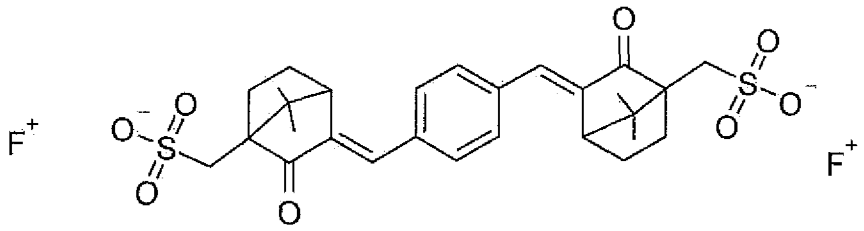

(I)

in which F denotes a hydrogen atom, an alkali metal or a radical $NH(R_1)_3^+$ in which the radicals $R_1$, which may be identical or different, denote a hydrogen atom, a $C_1$-$C_4$ alkyl or hydroxyalkyl radical or a group $M^{n+}/n$, $M^{n+}$ denoting a multivalent metal cation in which n is equal to 2 or 3 or 4, $M^{n+}$ preferably denoting a metal cation chosen from $Ca^{2+}$, $Zn^{2+}$, $Mg^{2+}$, $Ba^{2+}$, $Al^{3+}$ and $Zr^{4+}$.

**10.** Composition according to any one of Claims 1 to 5, in which the hydrophilic organic UVA-screening agent is a compound comprising at least two benzazolyl groups corresponding to the general formula (II) below:

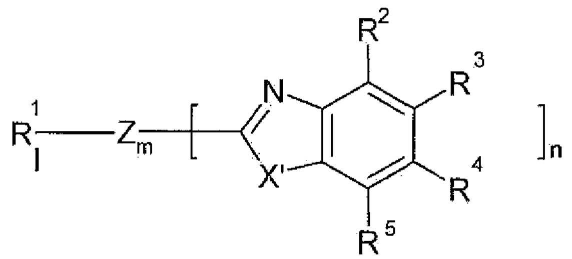

(II)

in which:

- Z represents an organic residue of valency (l + n) comprising one or more double bonds placed such that it completes the double bond system of at least two benzazolyl groups as defined within the brackets to form a fully conjugated assembly;
- X' denotes S, O or $NR^6$
- $R^1$ denotes hydrogen, $C_1$-$C_{18}$ alkyl, $C_1$-$C_4$ alkoxy, a $C_5$-$C_{15}$ aryl, a $C_2$-$C_{18}$ acyloxy, $SO_3Y$ or COOY;
- the radicals $R^2$, $R^3$, $R^4$ and $R^5$, which may be identical or different, denote a nitro group or a radical $R^1$;
- $R^6$ denotes hydrogen, a $C_1$-$C_4$ alkyl or a $C_1$-$C_4$ hydroxyalkyl;
- Y denotes hydrogen, Li, Na, K, $NH_4$, 1/2Ca, 1/2Mg, 1/3Al or a cation resulting from the neutralization of a free acid group with a nitrogenous organic base;
- m is 0 or 1;
- n is a number from 2 to 6;

- l is a number from 1 to 4;
- with the proviso that l + n does not exceed the value 6.

**11.** Composition according to Claim 10, in which the compound of formula (II) is chosen from those for which the group Z is chosen from the group consisting of:

(a) an olefinic linear aliphatic $C_2$-$C_6$ hydrocarbon-based radical which may be interrupted with a $C_5$-$C_{12}$ aryl group or a $C_4$-$C_{10}$ heteroaryl group, chosen in particular from:
-CH=CH-, -CH=CH-CH=CH- or

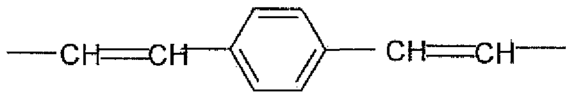

(b) a $C_5$-$C_{15}$ aryl group which may be interrupted with an olefinic linear aliphatic $C_2$-$C_6$ hydrocarbon-based radical chosen in particular from the following groups:

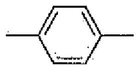

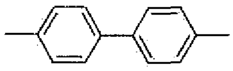

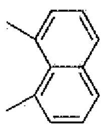

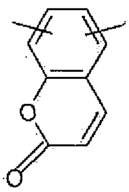

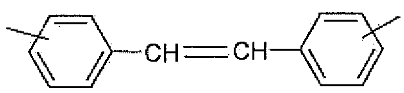

(c) a $C_3$-$C_{10}$ heteroaryl residue chosen in particular from the following groups:

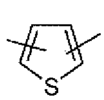 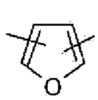 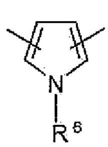 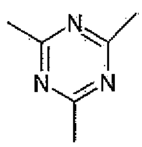

in which $R^6$ has the same meaning indicated above; the said radicals Z as defined in paragraphs (a), (b) and (c) possibly being substituted with $C_1$-$C_6$ alkyl, $C_1$-$C_6$ alkoxy, phenoxy, hydroxyl, methylenedioxy or amino radicals optionally substituted with one or two $C_1$-$C_5$ alkyl radicals.

**12.** Composition according to Claim 10 or 11, in which the compound of formula (II) comprises, per molecule, 1, 3 or 4 groups $SO_3Y$.

**13.** Composition according to any one of Claims 10 to 12, in which the compound of formula (II) is 1,4-bis(benzimidazolyl)phenylene-3,3',5,5'-tetrasulfonic acid having the following structure, or one of the salts thereof:

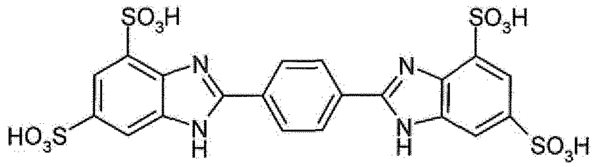

**14.** Composition according to any one of Claims 1 to 5, in which the hydrophilic organic UVA-screening agent is chosen from benzophenone compounds comprising at least one sulfonic acid function, and especially chosen from the following compounds:

Benzophenone-4,
Benzophenone-5,
Benzophenone-9, or mixtures thereof.

**15.** Composition according to any one of Claims 1 to 5, 9 to 14 in which the hydrophilic organic UVA-screening agent(s) are present in an active material concentration ranging from 1% to 10% by weight relative to the total weight of the composition.

**16.** Non-therapeutic cosmetic process for caring for and/or making up a keratin material, comprising the application to the surface of the said keratin material of at least one composition as defined in any one of the preceding claims.

**17.** Non-therapeutic cosmetic process for limiting the darkening of the skin and/or improving the colour and/or the uniformity of the complexion, comprising the application to the surface of the skin of at least one composition as defined in any one of the preceding claims.

**18.** Non-therapeutic cosmetic process for preventing and/or treating the signs of ageing of a keratin material, comprising the application to the surface of the keratin material of at least one composition as defined in any one of the preceding claims.

**Patentansprüche**

**1.** Kosmetische oder dermatologische Zusammensetzung, umfassend in einem physiologisch unbedenklichen Träger:

a) mindestens eine ölige Phase und
b) mindestens eine Merocyanin-Verbindung der nachstehenden Formel (1) sowie die geometrischen E/E- oder E/Z-Isomerformen davon:

(1) 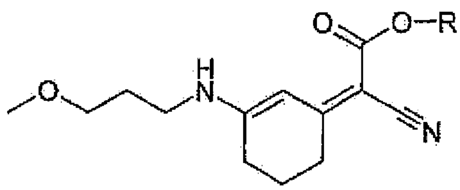

wobei:
R für eine $C_1$-$C_{22}$-Alkylgruppe, eine $C_2$-$C_{22}$-Alkenylgruppe, eine $C_2$-$C_{22}$-Alkinylgruppe, eine $C_3$-$C_{22}$-Cycloalkylgruppe oder eine $C_3$-$C_{22}$-Cycloalkenylgruppe steht, wobei die Gruppen gegebenenfalls durch ein oder mehrere O unterbrochen sein können, und
c) mindestens eine UV-A-Filtersubstanz, ausgewählt aus:

(i) einer aminosubstituierten 2-Hydroxybenzophenon-Verbindung und/oder
(ii) einer hydrophilen organischen UV-A-Filtersubstanz,
wobei die hydrophile organische UV-A-Filtersubstanz in einer Menge von 0,6 bis 15 Gew.-%, bezogen auf

das Gesamtgewicht der Zusammensetzung, vorliegt.

**2.** Zusammensetzung nach Anspruch 1, wobei die Merocyanin-Verbindung(en) der Formel (1) aus denjenigen ausgewählt sind, in denen:
R für ein $C_1$-$C_{22}$-Alkyl, das durch ein oder mehrere O unterbrochen sein kann, steht.

**3.** Zusammensetzung nach Anspruch 1 oder 2, wobei die Merocyanin-Verbindung(en) der Formel (1) aus den folgenden Verbindungen sowie den geometrischen E/E- oder E/Z-Isomerformen davon ausgewählt sind:

| | | | |
|---|---|---|---|
| 1 | 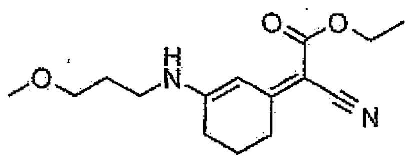<br>(2Z)-Cyano{3-[(3-methoxypropyl)amino]-cyclohex-2-en-1-yliden}ethansäure-ethylester | 4 | 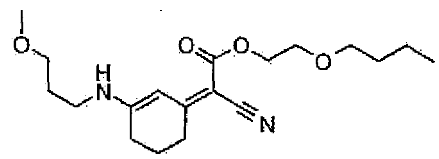<br>(2z)-Cyano{3-[(3-methoxypropyl)amino]-cyclohex-2-en-1-yliden}ethansäure-2-butoxyethylester |
| 2 | 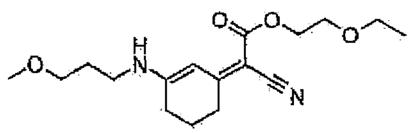<br>(2Z)-Cyano{3-[(3-methoxypropyl)amino]-cyclohex-2-en-1-yliden}ethansäure-2-ethoxyethylester | 5 | 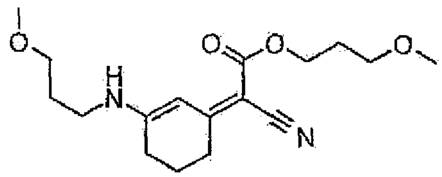<br>(2Z)-Cyano(3-[(3-methoxypropyl)amino]-cyclohex-2-en-1-yliden}ethansäure-3-methoxypropylester |
| 3 | 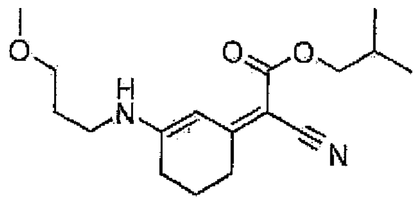<br>(2Z)-Cyano{3-[(3-methoxypropyl)amino]-cyclohex-2-en-1-yliden}ethansäure-2-methylpropylester | 6 | 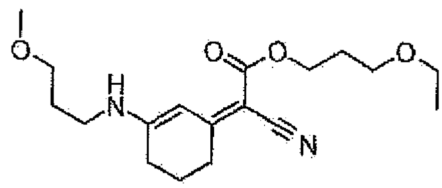<br>(2Z)-Cyano{3-[(3-methoxypropyl)amino]-cyclohex-2-en-1-yliden}ethansäure-3-ethoxypropylester |

**4.** Zusammensetzung nach Anspruch 3, wobei es sich bei der Merocyanin-Verbindung um (2Z)-Cyano{3-[(3-methoxypropyl)amino]cyclohex-2-en-1-yliden}ethansäure-2-ethoxyethylester (2) in seiner geometrischen E/Z-Konfiguration mit der folgenden Struktur:

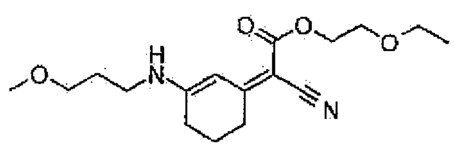

und/oder in seiner geometrischen E/E-Konfiguration mit der folgenden Struktur:

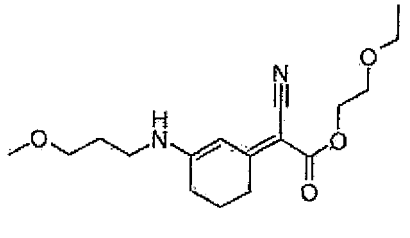

handelt.

5. Zusammensetzung nach einem der Ansprüche 1 bis 4, wobei die Merocyanin-Verbindung(en) der Formel (1) in einer Konzentration im Bereich von 0,1 bis 10 Gew.-% und vorzugsweise von 0,2 bis 5 Gew.-%, bezogen auf das Gesamtgewicht der Zusammensetzung, vorliegen.

6. Zusammensetzung nach einem der Ansprüche 1 bis 5, wobei die aminosubstituierte(n) 2-Hydroxybenzophenon-Verbindungen der nachstehenden Formel (I) entsprechen:

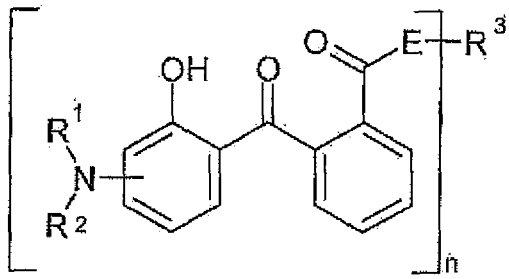

(I)

wobei:

$R^1$ und $R^2$, die gleich oder verschieden sein können, für einen $C_1$-$C_{20}$-Alkylrest, einen $C_2$-$C_{10}$-Alkenylrest, einen $C_3$-$C_{10}$-Cycloalkylrest oder einen $C_3$-$C_{10}$-Cycloalkenylrest stehen oder mit dem Stickstoffatom, an das sie gebunden sind, einen 5- oder 6-gliedrigen Ring bilden;
n für eine Zahl im Bereich von 1 bis 4 steht;
im Fall von n = 1 $R^3$ für einen $C_1$-$C_{20}$-Alkylrest, einen $C_2$-$C_{10}$-Alkenylrest, einen $C_2$-$C_5$-Hydroxyalkylrest, einen $C_6$-$C_{12}$-Cyclohexylrest, ein Phenyl, das durch O, N oder S substituiert sein kann, einen Aminocarbonylrest oder einen $C_1$-$C_5$-Alkylcarbonylrest steht;
im Fall von n = 2 $R^3$ für einen zweiwertigen Alkylrest, einen zweiwertigen Cycloalkylrest, einen zweiwertigen Alkenylrest oder einen zweiwertigen Arylrest steht oder $R^3$ mit E einen zweiwertigen Rest der Formel (II):

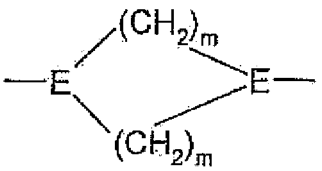

(II)

bildet, wobei m für eine ganze Zahl im Bereich von 1 bis 3 steht;
im Fall von n = 3 $R^3$ für einen dreiwertigen Alkylrest steht;
im Fall von n = 4 $R^3$ für einen vierwertigen Alkylrest steht;
E für -O-oder -N($R^4$)- oder N steht;
$R^4$ für Wasserstoff oder einen $C_1$-$C_5$-Alkyl- oder $C_1$-$C_5$-Hydroxyalkylrest steht.

7. Zusammensetzung nach Anspruch 6, wobei es sich bei der Verbindung der Formel (I) um 2-(4-Diethylamino-2-hydroxybenzoyl)benzoesäure-n-hexylester der Formel (a) :

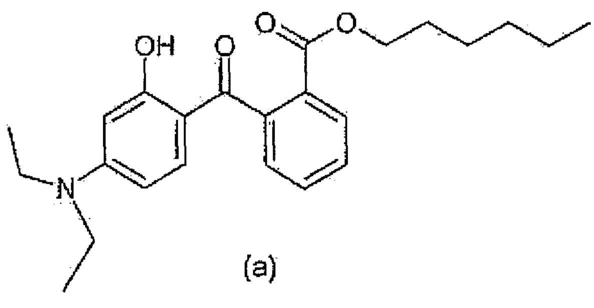

(a)

handelt.

**8.** Zusammensetzung nach Anspruch 6, wobei es sich bei der Verbindung der Formel (I) um (2-{4-[2-(4-Diethylamino-2-hydroxybenzoyl)benzoyl]piperazin-1-carbonyl}phenyl) (4-diethylamino-2-hydroxyphenyl)-methanon der Formel (b):

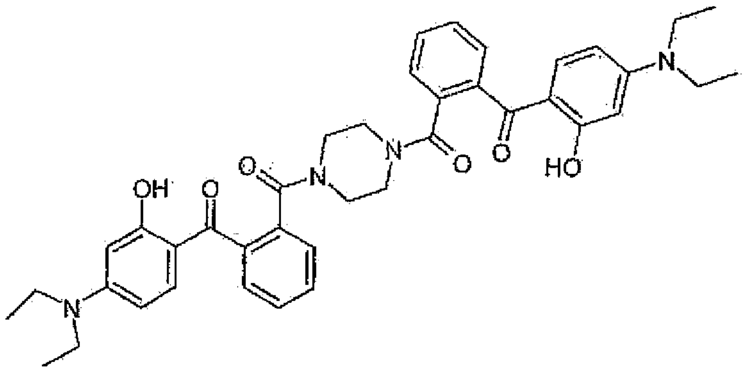

(b)

handelt.

**9.** Zusammensetzung nach einem der Ansprüche 1 bis 5, wobei es sich bei der hydrophilen organischen UV-A-Filtersubstanz um Benzol-1,4-di(3-methyliden-10-camphersulfonsäure) oder eines der Salze davon sowie die geometrischen Isomere davon, die der folgenden allgemeinen Formel (I) entsprechen, handelt:

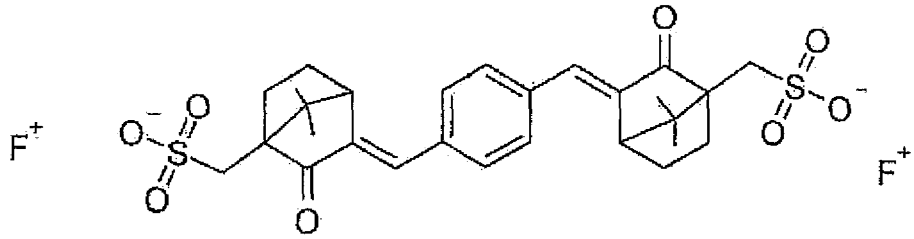

(I)

wobei F für ein Wasserstoffatom, ein Alkalimetall oder einen Rest $NH(R_1)_3^+$ steht, wobei die Reste $R_1$, die gleich oder verschieden sein können, für ein Wasserstoffatom, einen $C_1$-$C_4$-Alkyl- oder - Hydroxyalkylrest oder eine Gruppe $M^{n+}/n$ stehen, wobei $M^{n+}$ für ein mehrwertiges Metallkation steht, wobei n gleich 2 oder 3 oder 4 ist, wobei $M^{n+}$ vorzugsweise für ein aus $Ca^{2+}$, $Zn^{2+}$, $Mg^{2+}$, $Ba^{2+}$, $Al^{3+}$ und $Zr^{4+}$ ausgewähltes Metallkation steht.

**10.** Zusammensetzung nach einem der Ansprüche 1 bis 5, wobei es sich bei der hydrophilen organischen UV-A-Filtersubstanz um eine Verbindung mit mindestens zwei Benzazolylgruppen, die der nachstehenden allgemeinen Formel (II) entspricht, handelt:

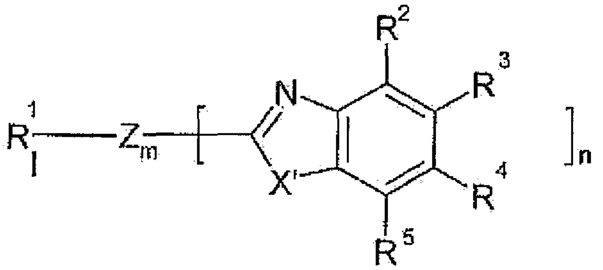

(II)

wobei:

- Z für einen organischen Rest mit der Wertigkeit (m + n) mit mindestens zwei Doppelbindungen, die so angeordnet sind, dass sie das Doppelbindungssystem von mindestens zwei Benzazolylgruppen, wie sie in den Klammern definiert sind, zur Bildung einer vollständig konjugierten Anordnung ergänzen;

- X' für S, O oder $NR^6$ steht;
- $R^1$ für Wasserstoff, $C_1$-$C_{18}$-Alkyl, $C_1$-$C_4$-Alkoxy, ein $C_5$-$C_{15}$-Aryl, ein $C_2$-$C_{18}$-Acyloxy, $SO_3Y$ oder COOY steht;
- die Reste $R^2$, $R^3$, $R^4$ und $R^5$, die gleich oder verschieden sein können, für eine Nitrogruppe oder einen Rest $R^1$ stehen;
- $R^6$ für Wasserstoff, ein $C_1$-$C_4$-Alkyl oder ein $C_1$-$C_4$-Hydroxyalkyl steht;
- Y für Wasserstoff, Li, Na, K, $NH_4$, 1/2Ca, 1/2Mg, 1/3A1 oder ein Kation, das sich aus der Neutralisation einer freien Säuregruppe mit einer stickstoffhaltigen organischen Base ergibt, steht;
- m für 0 oder 1 steht;
- n für eine ganze Zahl von 2 bis 6 steht;
- l für eine ganze Zahl von 1 bis 4 steht;
- mit der Maßgabe, dass l + n den Wert 6 nicht überschreitet.

**11.** Zusammensetzung nach Anspruch 10, wobei die Verbindung der Formel (II) aus denjenigen ausgewählt ist, für die die Gruppe Z ausgewählt ist aus der Gruppe bestehend aus:

(a) einem olefinischen linearen aliphatischen $C_2$-$C_6$-Rest auf Kohlenwasserstoffbasis, der durch eine $C_5$-$C_{12}$-Arylgruppe oder eine $C_4$-$C_{10}$-Heteroarylgruppe unterbrochen sein kann, insbesondere ausgewählt aus: -CH=CH- -CH=CH-CH=CH- oder

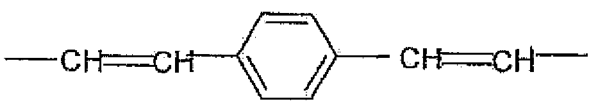

[or -> oder]

(b) einer $C_5$-$C_{15}$-Arylgruppe, die durch einen olefinischen linearen aliphatischen $C_2$-$C_6$-Rest auf Kohlenwasserstoffbasis unterbrochen sein kann, insbesondere ausgewählt aus den folgenden Gruppen:

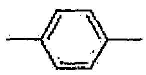

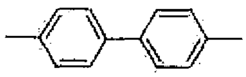

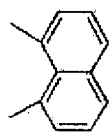

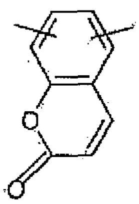

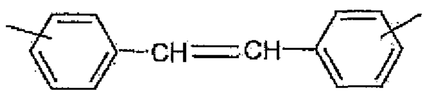

(c) einem $C_3$-$C_{10}$-Heteroarylrest, insbesondere ausgewählt aus den folgenden Gruppen:

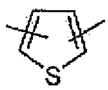 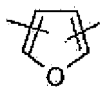 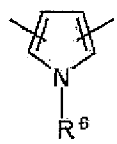 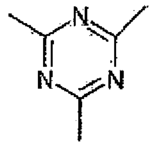

wobei $R^6$ die gleiche Bedeutung wie oben angegeben hat; wobei die Reste Z, wie sie in den Absätzen (a), (b) und (c) definiert sind, gegebenenfalls durch $C_1$-$C_6$-Alkyl-, $C_1$-$C_6$-Alkoxy-, Phenoxy-, Hydroxyl-, Methylendioxy- oder Aminoreste, die gegebenenfalls durch einen oder zwei $C_1$-$C_5$-Alkylreste substituiert sind, substituiert sind.

**12.** Zusammensetzung nach Anspruch 10 oder 11, wobei die Verbindung der Formel (II) pro Molekül 1, 3 oder 4 Gruppen $SO_3Y$ umfasst.

**13.** Zusammensetzung nach einem der Ansprüche 10 bis 12, wobei es sich bei der Verbindung der Formel (II) um 1,4-Bis(benzimidazolyl)phenylen-3,3',5,5'-tetrasulfonsäure mit der folgenden Struktur oder eines der Salze davon handelt:

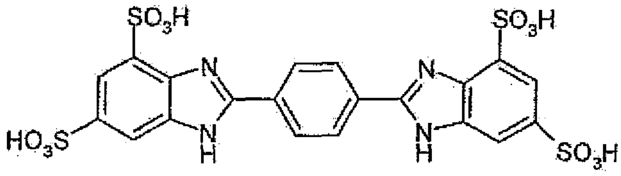

**14.** Zusammensetzung nach einem der Ansprüche 1 bis 5, wobei die hydrophile organische UV-A-Filtersubstanz aus Benzophenon-Verbindungen mit mindestens einer Sulfonsäurefunktion ausgewählt ist und insbesondere aus den folgenden Verbindungen ausgewählt ist:

Benzophenone-4,
Benzophenone-5,
Benzophenone-9 oder Mischungen davon.

**15.** Zusammensetzung nach einem der Ansprüche 1 bis 5,9 bis 14, wobei die hydrophile(n) organische(n) UV-A-Filtersubstanz(en) in einer Wirkstoffkonzentration von 1 bis 10 Gew.-%, bezogen auf das Gesamtgewicht der Zusammensetzung, vorliegen.

**16.** Nichttherapeutisches kosmetisches Verfahren zum Pflegen und/oder Schminken eines Keratinmaterials, bei dem man mindestens eine Zusammensetzung gemäß einem der vorhergehenden Ansprüche auf die Oberfläche des Keratinmaterials aufbringt.

**17.** Nichttherapeutisches kosmetisches Verfahren zum Einschränken des Dunkelwerdens der Haut und/oder zum Verbessern der Farbe und/oder Einheitlichkeit des Teints, bei dem man mindestens eine Zusammensetzung gemäß einem der vorhergehenden Ansprüche auf die Oberfläche der Haut aufbringt.

**18.** Nichttherapeutisches kosmetisches Verfahren zum Verhindern und/oder Behandeln der Anzeichen von Alterung eines Keratinmaterials, bei dem man mindestens eine Zusammensetzung gemäß einem der vorhergehenden Ansprüche auf die Oberfläche des Keratinmaterials aufbringt.

## Revendications

**1.** Composition cosmétique ou dermatologique comprenant, dans un support physiologiquement acceptable :

a) au moins une phase huileuse et
b) au moins un composé mérocyanine répondant à la formule (1) suivante, ainsi que ses formes géométriques isomères E/E- ou E/Z- :

(1) 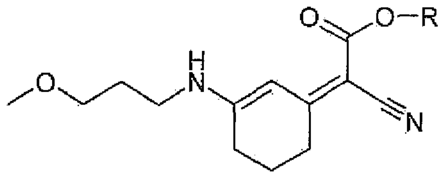

dans laquelle

R est un groupe alkyle en $C_1$-$C_{22}$, un groupe alcynyle en $C_2$-$C_{22}$, un groupe alcynyle en $C_2$-$C_{22}$, un groupe cycloalkyle en $C_3$-$C_{22}$ ou un groupe cycloalcényle en $C_3$-$C_{22}$, lesdits groupes pouvant être interrompus par un ou plusieurs O et

c) au moins un filtre UVA choisi parmi :

(i) un composé 2-hydroxybenzophénone amino-substitué et/ou

(ii) un filtre UVA organique hydrophile ; et ledit filtre UVA organique hydrophile étant présent dans une quantité allant de 0,6% à 15 % en poids par rapport au poids total de la composition.

**2.** Composition selon la revendication 1, où le ou les composés mérocyanine de formule (1) sont choisis parmi ceux où R est un alkyle en $C_1$-$C_{22}$, pouvant être interrompu par un ou plusieurs O.

**3.** Composition selon la revendication 1 ou 2, où le ou les composés mérocyanine de formule (1) sont choisis parmi les composés suivants, ainsi que leurs formes géométriques isomères E/E- ou E/Z- :

| | | | |
|---|---|---|---|
| 1 | 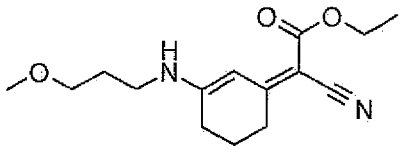<br>éthyl (2Z)-cyano{3-[(3-méthoxypropyl) amino]-cyclohex-2-én-1-ylidène}éthanoate | 4 | 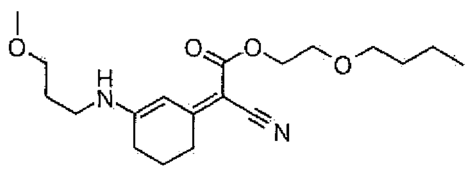<br>2-butoxyéthyl (2Z)-cyano{3-[(3-méthoxypropyl) amino]-cyclohex-2-én-1-ylidène}éthanoate |
| 2 | 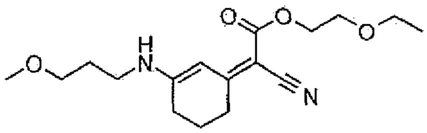<br>2-éthoxyethyl (2Z)-cyano{3-[(3-méthoxypropyl) amino]-cyclohex-2-én-1-ylidène}éthanoate | 5 | 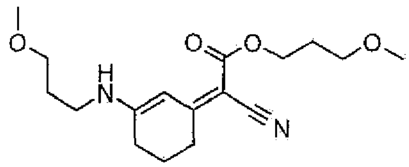<br>3-méthoxypropyl (2Z)-cyano{3-[(3-méthoxypropyl) amino]-cyclohex-2-én-1-ylidène}éthanoate |
| 3 | 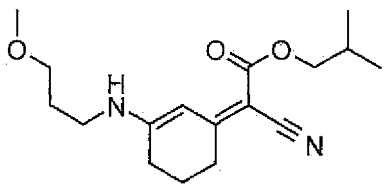<br>2-méthylpropyl (2Z)-cyano{3-[(3-méthoxypropyl) amino]-cyclohex-2-én-1-ylidène}éthanoate | 6 | 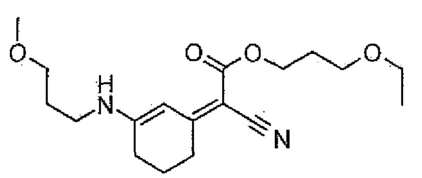<br>3-éthoxypropyl (2Z)-cyano{3-[(3-méthoxypropyl)-amino]cyclohex-2-én-1-ylidène} éthanoate |

**4.** Composition selon la revendication 3, où le composé mérocyanine est le 2-éthoxyéthyle (2Z)-cyano{3-[(3-méthoxypropyl)amino]cyclohex-2-én-1-ylidène}éthanoate (2) dans sa configuration géométrique E/Z de structure suivante :

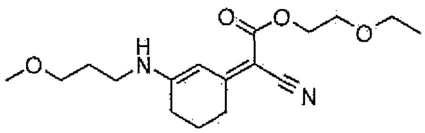

et/ou dans sa configuration géométrique E/E de structure suivante :

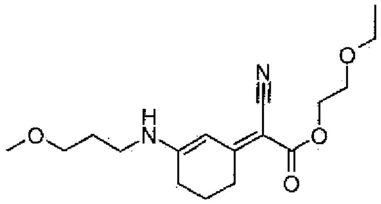

**5.** Composition selon l'une quelconque des revendications 1 à 4, où le ou les composés mérocyanine de formule (1) sont présents dans une concentration allant de 0,1 % à 10 % en poids, et préférentiellement de 0,2 % à 5 % en poids par rapport au poids total de la composition.

**6.** Composition selon l'une quelconque des revendications 1 à 5, où le ou les composés 2-hydroxybenzophénone amino- substitués répondent à la formule (I) suivante :

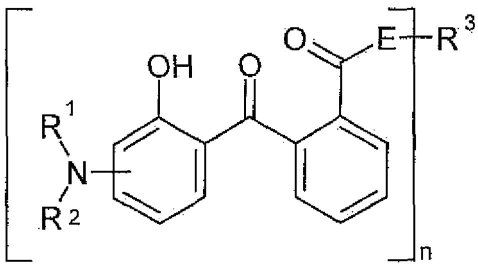

(I)

dans laquelle :

$R^1$ et $R^2$, identiques ou différents, désignent un radical alkyle en $C_1$-$C_{20}$, un radical alcényle en $C_2$-$C_{20}$, un radical cycloalkyle en $C_3$-$C_{10}$ ou un radical cycloalcényle en $C_3$-$C_{10}$ ou forment avec l'atome d'azote avec lequel ils sont liés un cycle à 5 ou 6 chaînons ;
n est un nombre allant de 1 à 4 ;
quand n = 1, $R^3$ désigne un radical alkyle en $C_1$-$C_{20}$, un radical alcényle en $C_1$-$C_{20}$, un radical hydroxyalkyle en $C_1$-$C_5$, un radical cyclohexyle en $C_6$-$C_{12}$, un phényle pouvant être substitué par O, N ou S, un radical aminocarbonyle ou un radical alkylcarbonyle en $C_1$-$C_5$ ;
quand n = 2, $R^3$ désigne un diradical alkyle, un diradical cycloalkyle, un diradical alcényle ou un diradical aryle ou $R^3$ avec E forment un diradical de formule (II) :

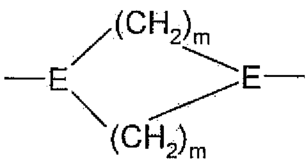

(II)

avec m un nombre allant de 1 à 3 ;
quand n = 3, $R^3$ est un triradical alkyle ;
quand n = 4 , $R^3$ est un tétraradical alkyle ;
E est -O-, ou -N($R^4$)- ou N ;
$R^4$ est hydrogène, un radical alkyle en $C_1$-$C_5$ ou hydroxyalkyle en $C_1$-$C_5$.

**7.** Composition selon la revendication 6, où le composé de formule (I) est le 2-(4-diéthylamino-2-hydroxybenzoyl)benzoate de n-hexyle de formule (a) :

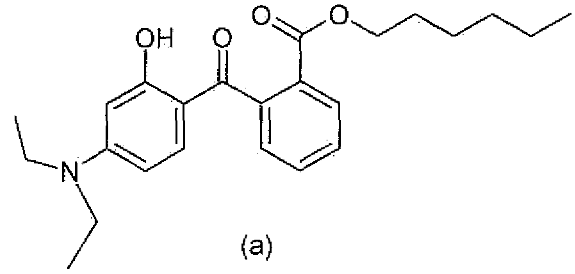

(a)

**8.** Composition selon la revendication 6, où le composé de formule (I) est le (2-{4-[2-(4-diéthylamino-2-hydroxybenzoyl)benzoyl]pipérazine-1-carbonyl}phényl)-(4-diéthylamino-2-hydroxyphenyl)méthanone de formule (b) :

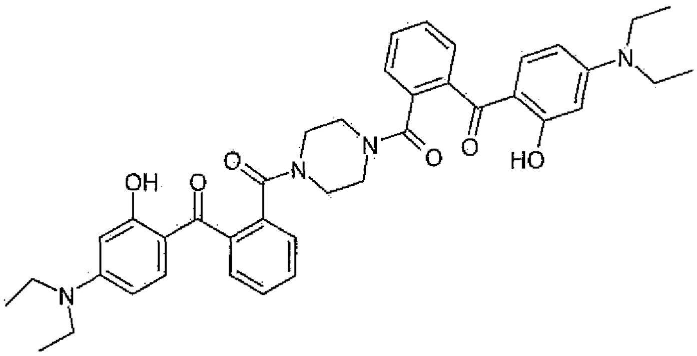

(b)

**9.** Composition selon l'une quelconque des revendications 1 à 5, où le filtre UVA organique hydrophile est l'acide benzène 1,4-bis(3-méthylidène-10-camphosulfonique) ou l'un de ses sels ainsi que ses isomères géométriques répondant à la formule générale (I) suivante :

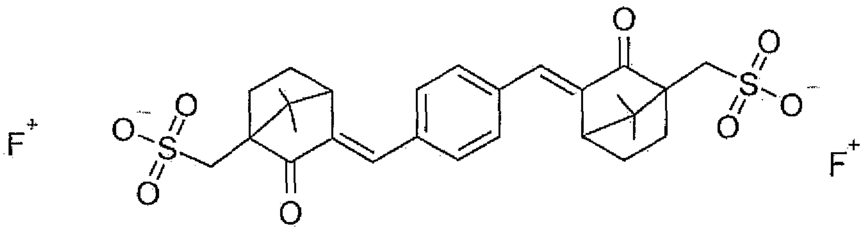

(I)

dans laquelle F désigne un atome d'hydrogène, un métal alcalin ou un radical NH $(R_1)_3^+$ dans lequel les radicaux $R_1$, qui peuvent être identiques ou différents, désignent un atome d'hydrogène, un radical alkyle ou hydroxyalkyle en $C_1$-$C_4$ ou un groupe $M^{n+}/n$, $M^{n+}$ désignant un cation métallique polyvalent dans lequel n est égal à 2 ou 3 ou 4, $M^{n+}$ désignant de préférence un cation métallique choisi parmi $Ca^{2+}$, $Zn^{2+}$, $Mg^{2+}$, $Ba^{2+}$, $Al^{3+}$ et $Zr^{4+}$.

**10.** Composition selon l'une quelconque des revendications 1 à 5, où le filtre UVA organique hydrophile est un composé comportant au moins deux groupes benzoazolyle répondant à la formule générale (II) suivante :

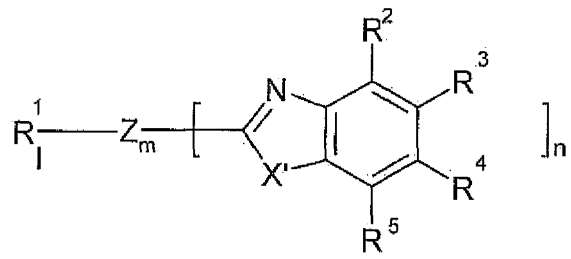

(II)

dans laquelle :

- Z représente un reste organique de valence (1 + n) comportant une ou plusieurs doubles liaisons placées de telle sorte qu'elle complète le système de doubles liaisons d'au moins deux groupes benzoazolyle tels que définis à l'intérieur des crochets pour former un ensemble totalement conjugué ;
- X' désigne S, O ou $NR^6$
- $R^1$ désigne l'hydrogène, un alkyle en $C_1$-$C_{18}$, un alcoxy en $C_1$-$C_4$, un aryle en $C_5$-$C_{15}$, un acyloxy en $C_2$-$C_{18}$, $SO_3Y$ ou COOY ;
- les radicaux $R^2$, $R^3$, $R^4$ et $R^5$, identiques ou différents, désignent un groupe nitro ou un radical $R^1$ ;
- $R^6$ désigne l'hydrogène, un alkyle en $C_1$-$C_4$ ou un hydroxyalkyle en $C_1$-$C_4$ ;
- Y désigne l'hydrogène, Li, Na, K, $NH_4$, 1/2Ca, 1/2Mg, 1/3Al ou un cation résultant de la neutralisation d'un groupe acide libre par une base azotée organique ;
- m est 0 ou 1 ;
- n est un nombre de 2 à 6 ;
- l est un nombre de 1 à 4 ;
- sous réserve que 1 + n ne dépasse pas la valeur 6.

**11.** Composition selon la revendication 10, où le composé de formule (II) est choisi parmi ceux pour lesquels le groupe Z est choisi dans le groupe constitué par :

(a) un radical hydrocarboné en $C_2$-$C_6$ aliphatique linéaire oléfine pouvant être interrompu par un groupe aryle en $C_5$-$C_{12}$ ou un groupe hétéroaryle en $C_4$-$C_{10}$ en particulier choisi parmi : -CH=CH-, -CH=CH-CH=CH- ou bien

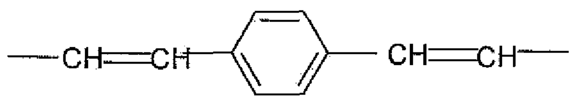

(b) un groupe aryle en $C_5$-$C_{15}$ pouvant être interrompu par un radical hydrocarboné en $C_2$-$C_6$ aliphatique linéaire oléfine en particulier choisi parmi les groupes suivants :

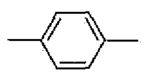

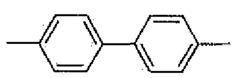

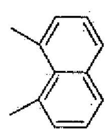

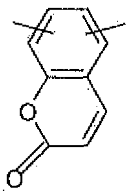

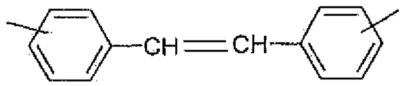

(c) un reste hétéroaryle en C3-$C_{10}$, en particulier choisi parmi les groupes suivants :

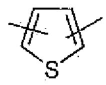 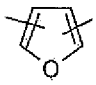 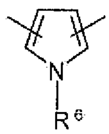 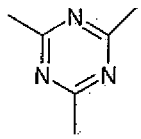

où $R^6$ a la même signification indiquée ci-dessus ; lesdits radicaux Z tels que définis dans les paragraphes (a), (b) et (c) pouvant être substitués par des radicaux alkyle en $C_1$-$C_6$, alcoxy en $C_1$-$C_6$, phénoxy, hydroxy, méthylènedioxy ou amino éventuellement substitués par un ou deux radicaux alkyle en $C_1$-$C_5$.

**12.** Composition selon la revendication 10 ou 11, où le composé de formule (II) comporte par molécule 1, 3 ou 4 groupes $SO_3Y$.

**13.** Composition selon l'une quelconque des revendications 10 à 12, où le composé de formule (II) est l'acide 1,4-bis(benzimidazolyl)phènylène-3,3',5,5'-tétrasulfonique de structure suivante ou l'un de ses sels :

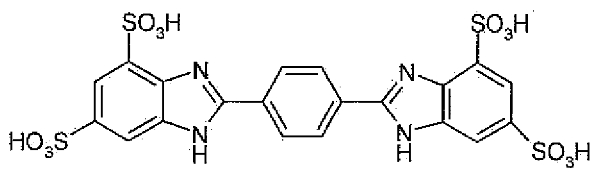

**14.** Composition selon l'une quelconque des revendications 1 à 5, où le filtre UVA organique hydrophile est choisi parmi les composés benzophénone comprenant au moins une fonction acide sulfonique, et notamment choisi parmi les composés suivants :

Benzophénone-4,
Benzophénone-5,
Benzophénone-9 ou leurs mélanges.

**15.** Composition selon l'une quelconque des revendications 1 à 5, 9 à 14 où le filtre ou les filtres UVA organiques hydrophiles sont présents dans une concentration en matière active allant de 1 % à 10 % en poids par rapport au poids total de la composition.

**16.** Procédé cosmétique non-thérapeutique de soin et/ou de maquillage d'une matière kératinique comprenant l'application, sur la surface de ladite matière kératinique, d'au moins une composition telle que définie dans l'une quelconque des revendications précédentes.

**17.** Procédé cosmétique non-thérapeutique pour limiter l'assombrissement de la peau et/ou améliorer la couleur et/ou l'homogénéité du teint comprenant l'application, sur la surface de la peau, d'au moins une composition telle que définie dans l'une quelconque des revendications précédentes.

**18.** Procédé cosmétique non-thérapeutique pour prévenir et/ou traiter les signes du vieillissement d'une matière kératinique comprenant l'application, sur la surface de la matière kératinique, d'au moins une composition telle que définie dans l'une quelconque des revendications précédentes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1046391 A
- EP 1133980 A
- DE 10012408
- WO 2007071584 A
- FR 2528420 A
- FR 2639347 A
- EP 0669323 A
- US 2463264 A
- US 4195999 A
- WO 2004006878 A
- WO 2008090066 A
- WO 2011113718 A
- WO 2009027258 A
- WO 2013010590 A
- WO 2013011094 A
- WO 2013011480 A
- WO 2007071582 A
- US 4749643 A
- GB 2303549 A
- EP 893119 A
- WO 2007068371 A
- WO 2008155059 A
- US 5624663 A
- EP 669323 A
- US 5237071 A
- US 5166355 A
- DE 19726184
- EP 0832642 A
- EP 1027883 A
- EP 1300137 A
- DE 10162844
- WO 9304665 A
- DE 19855649
- EP 0967200 A
- DE 19746654
- DE 19755649
- EP 1008586 A
- EP 133981 A
- WO 2009063392 A
- US 6225467 B
- WO 2004085412 A
- WO 06035000 A
- WO 06034982 A
- WO 06034991 A
- WO 06035007 A
- WO 2006034992 A
- WO 2006034985 A
- EP A A
- EP 0841341 A
- EP 0518773 A
- WO 9206778 A
- FR 2315991
- FR 2416008
- US 4077441 A
- US 4850517 A


### Non-patent literature cited in the description


- *IP COM JOURNAL N°000179675D,* 23 February 2009
- *IP COM JOURNAL N°000182396D*
- *IP COM JOURNAL N° 000189542D,* 12 November 2009
- *IP COM Journal N°IPCOM000011179D,* 03 April 2004
- *IP.com Journal,* 2009, vol. 9 (5A), 29-30
- Symmetrical Triazine Derivatives. IP.COM IPCOM000031257 Journal, INC, 20 September 2004
- HLB Index. McCutcheon's Emulsifiers & Detergents. MC Publishing Company, 1998
- **BANGHAM ; STANDISH ; WATKINS.** *J. Mol. Biol.,* 1965, vol. 13, 238
- **B.L. DIFFEY.** *J. Soc. Cosmet. Chem.,* 1989, vol. 40, 127-133